# FASCICULE DE BREVET EUROPEEN

(11) **EP 2 300 405 B1**
(45) Date de publication et mention de la délivrance du brevet: **07.09.2016**
(21) Numéro de dépôt: 09745984.6
(22) Date de dépôt: 23.04.2009
(51) Int. Cl.: C07C 43/247, C07C 43/253, C07C 47/277, C07C 229/36, C07C 227/32

(54) **PROCÉDÉS DE SYNTHÈSE ASYMÉTRIQUE DE LA 6-FLUORO-L-DOPA ET DE SES ANALOGUES**
VERFAHREN ZUR ASYMMETRISCHEN SYNTHESE VON 6-FLUOR-L-DOPA UND ANALOGA DAVON
METHODS FOR ASYMMETRICALLY SYNTHESIZING 6-FLUORO-L-DOPA AND ANALOGS THEREOF

(30) Priorité: 28.04.2008 FR 0852859
(43) Date de publication de la demande: 30.03.2011
(73) Titulaire: Laboratoires Cyclopharma, 63360 Saint Beauzire (FR); Centre National de la Recherche Scientifique (CNRS), 75016 Paris (FR); Université Paul Sabatier Toulouse III, 31062 Toulouse Cedex 9 (FR)
(72) Inventeur: MARETTE, Caroline, F-14610 Cairon (FR); GRAS, Emmanuel, F-31860 Pins Justaret (FR)
(74) Mandataire: Lavoix
(86) Numéro de dépôt international: PCT/FR2009/050752
(87) Numéro de publication internationale: WO 2009/138665

(56) Documents cités:
- CHRISTIAN LEMAIRE ET AL: "Highly enantioselective synthesis of No-carrier-added 6-(18F)fluoro-L-dopa by chiral phase-transfer alkylation" EUROPEAN JOURNAL OF ORGANIC CHEMISTRY., no. 13, 2004, pages 2899-2904, XP002509250 DEWILEY-VCH, WEINHEIM. cité dans la demande
- LEMAIRE C ET AL: "An approach to the asymmetric synthesis of l-6-[<18>F]fluorodopa via NCA nucleophilic fluorination" APPLIED RADIATION AND ISOTOPES, INTERNATIONAL JOURNAL OFRADIATION APPLICATIONS AND INSTRUMENTATION, PART A, PERGAMON PRESS LTD, GB, vol. 42, no. 7, 1 janvier 1991 (1991-01-01), pages 629-635, XP024706741 ISSN: 0883-2889 [extrait le 1991-01-01] cité dans la demande
- DATABASE CA [Online] CHEMICAL ABSTRACTS SERVICE, COLUMBUS, OHIO, US; ZHANG, LAN ET AL: "Preparation of 6-[F18]fluoro-L-dopa" XP002509251 extrait de STN Database accession no. 2005:391357 & CN 1 435 409 A (SHANGHAI INSTITUTE OF ATOMIC NUCLEUS, CHINESE ACADEMY OF SCIENCES, PEO) 13 août 2003 (2003-08-13)
- ZHANG L ET AL: "Enantioselective synthesis of no-carrier-added (NCA) 6-[<18>F]fluoro-l-DOPA" APPLIED RADIATION AND ISOTOPES, ELSEVIER, OXFORD, GB, vol. 57, no. 2, 1 août 2002 (2002-08-01), pages 145-151, XP004361141 ISSN: 0969-8043
- LEMAIRE C ET AL: "ENANTIOSELECTIVE SYNTHESIS OF 6-ÄFLUORINE-18Ü-FLUORO-L-DOPA FROM NO-CARRIER-ADDED FLUORINE-18-FLUORIDE" JOURNAL OF NUCLEAR MEDICINE, SOCIETY OF NUCLEAR MEDICINE, RESTON, VA, US, vol. 35, no. 12, 1 décembre 1994 (1994-12-01), pages 1996-2002, XP008072789 ISSN: 0161-5505
- LEMAIRE C ET AL: "Nucleophilic enantioselective synthesis of 6-[<18>F]Fluoro-l-dopa via two chiral auxiliaries" APPLIED RADIATION AND ISOTOPES, ELSEVIER, OXFORD, GB, vol. 44, no. 4, 1 avril 1993 (1993-04-01), pages 737-744, XP002255569 ISSN: 0969-8043 [extrait le 1993-04-01]
- DING Y-S ET AL: "No-Carrier-Added (NCA) {18F}Fluorides via the Nucleophilic Aromatic Substitution of Electron-Rich Aromatic Rings" JOURNAL OF FLUORINE CHEMISTRY, ELSEVIER, NL, vol. 48, no. 2, 1 janvier 1990 (1990-01-01), pages 189-205, XP002215522 ISSN: 0022-1139

## Description

L'invention concerne un procédé de synthèse énantiosélectif, d'une part, et diastéréosélectif, d'autre part, de la 6-fluoro-L-Dopa et de ses analogues, notamment radiomarquée au ¹⁸F.

En 1983, Garnett et coll. ont, pour la première fois, mis en évidence que la 6-[¹⁸F]fluoro-L-Dopa pouvait être le radiotraceur présynaptique idéal pour donner le reflet de la distribution cérébrale de la dopamine et de son métabolisme, d'abord chez le singe (E.S. Garnett et al., Brain Res., 1983) puis chez l'homme (E.S. Garnett et al., Nature, 1983). Du fait de l'implication de ce neurotransmetteur dans des maladies neurodégénératives de type Parkinson, il apparaissait que le recours à la 6-[¹⁸F]fluoro-L-Dopa pourrait être l'atout majeur pour la compréhension de ces pathologies. Il s'ensuit alors un grand nombre de publications dédiées à la synthèse de ce radiopharmaceutique, dont notamment des synthèses asymétriques, lesquelles permettent de s'affranchir de l'étape de séparation des énantiomères, coûteuse en temps et donc en produit radiomarqué.

Deux voies ont été développées :
- une approche diastéréosélective utilisant une copule chirale ;
- une approche reposant sur une catalyse énantiosélective.

Les synthèses diastéréosélectives de la 6-[¹⁸F]fluoro-L-Dopa (4) ont été réalisées en présence de différentes copules chirales.

Une de ces approches utilise comme copule chirale l'ester tertiobutylique de (1 R, 2R,5R)-[(+)-2-hydroxy-pinanyl-3-idène]glycine B optiquement pur (C. Lemaire et al., J. Nucl. Med., 1989; C. Lemaire et al., Appl. Radiat. Isot., 1991).

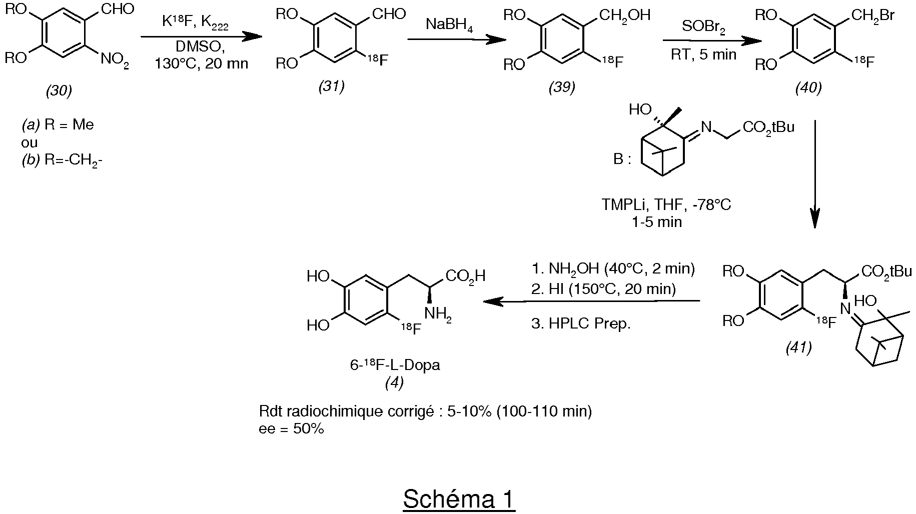

Toutefois, l'excès énantiomérique obtenu est faible et ne permet naturellement pas de se passer d'une purification finale sur colonne chirale ne donnant à cette synthèse qu'un intérêt conceptuel.

Une synthèse énantiosélective de la 6-[¹⁸F]-fluoro-L-Dopa a récemment été décrite (C. Lemaire et al., Eur. J. Org. Chem., 2004) :

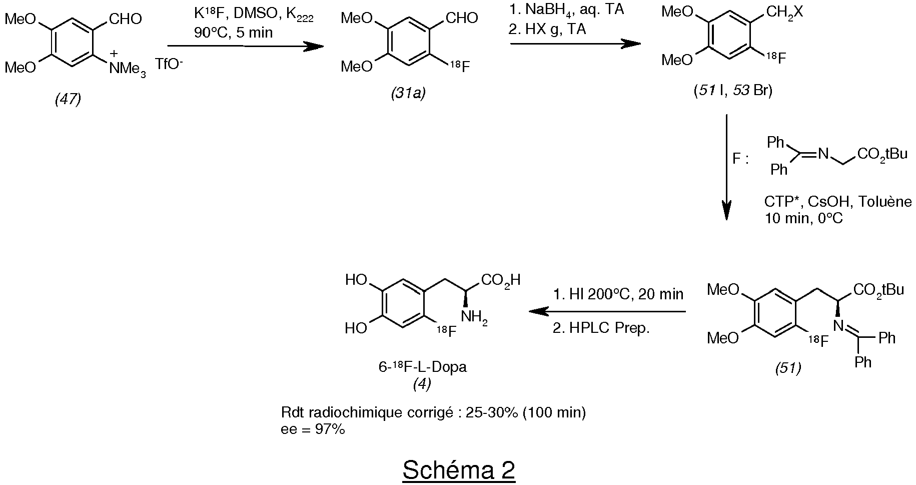

Ce procédé met en oeuvre une alkylation énantiosélective utilisant le catalyseur de transfert de phase chiral CTP* de structure suivante :

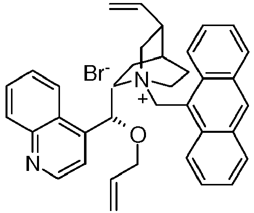

Ce procédé permet l'obtention de rendements radiochimiques corrigés et d'excès énantiomériques satisfaisants.

Cependant, ces deux approches requièrent dans la dernière étape des conditions drastiques (HI, 200°C) pour déprotéger les fonctions alcool et ne permettent donc pas une automatisation aisée de la synthèse de la 6-[¹⁸F]-fluoro-L-Dopa.

Il a maintenant été mis au point un procédé de synthèse énantiosélective d'une part, et diastéréosélective d'autre part, de la 6-[¹⁸F]-fluoro-L-Dopa et de ses analogues avec des rendements radiochimiques et des excès énantiomériques (ee) élevés, notamment des ee supérieurs à 90%. En outre, ces procédés mettent en oeuvre des conditions relativement douces ce qui permet une automatisation robuste. Plus précisément, il a été découvert qu'en substituant les groupements méthyle protégeant les fonctions alcool du catéchol par des groupements protecteurs particuliers tels que le groupe benzoyle, il était possible d'obtenir à la fois une protection efficace des fonctions alcool, notamment en milieu basique aqueux, et une déprotection dans des conditions beaucoup plus douces que celles décrites dans l'art antérieur.

En outre, de par cette automatisation, ces procédés permettent de synthétiser la 6-[¹⁸F]-fluoro-L-Dopa ou ses analogues dans de meilleures conditions de sécurité en réduisant l'exposition à la radioactivité.

Ainsi, selon un premier objet, l'invention concerne des composés de formule (II) : 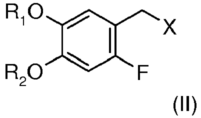 dans laquelle :
F est le ¹⁹F ou le ¹⁸F ;
R₁ représente un groupe aroyle ;
R₂ représente un groupe aroyle ;
X est Cl, Br, I, ou un groupe OSO₂R₃ ;
R₃ représente un groupe C₁-C₆ alkyle, ledit groupe alkyle étant éventuellement mono-, poly- ou perhalogéné ; un groupe C₃-C₈ cycloalkyle ou un groupe C₆-C₁₂ aryle, lesdits groupes alkyle, cycloalkyle, ou aryle étant substitués par F, Cl, Br, NO₂ ou CN.

Ces composés sont particulièrement utiles pour la mise en oeuvre des procédés de synthèse énantiosélective et/ou diastéréosélective selon l'invention.

Tels qu'on les utilise ci-dessus, et dans toute la description de l'invention, les termes suivants, sauf mention contraire, doivent être compris comme ayant les significations suivantes :
« Acyle » désigne un groupe alkyl-CO-, dans lequel le groupe alkyle est tel que décrit dans le présent document. Les groupes acyle préférés contiennent un alkyle inférieur. Comme exemples de groupe acyle, on peut citer notamment l'acétyle (CH₃-CO-), et le t-butanoyle ((CH₃)₃C-CO-).
« Alcényle » désigne un groupe hydrocarboné aliphatique qui contient une double liaison carbone-carbone et qui peut être linéaire ou ramifié ayant 2 à 15 atomes de carbone dans la chaîne. Les groupes alcényle préférés ont 2 à 12 atomes de carbone dans la chaîne, et de préférence 2 à 4 atomes de carbone dans la chaîne. «Ramifié » signifie qu'un ou plusieurs groupes alkyle inférieurs, tels que le méthyle, l'éthyle ou le propyle, sont liés à une chaîne alcényle linéaire. Comme exemple de groupe protecteur alcényle, on peut citer notamment le groupement alkyle (CH₂CH=CH₂).
« Alcoxyalkyle » désigne un groupe alkyl-O-alkyl-, dans lequel les groupes alkyle sont, indépendamment l'un de l'autre, tels que décrits dans le présent document. Comme exemple de groupe protecteur alcoxyalkyle, on peut citer notamment le méthoxyméthyle (-CH₂OCH₃).
« Alcoxyalcoxyalkyle » désigne un groupe alkyl-O-alkyl-O-alkyl-, dans lequel les groupes alkyle sont, indépendamment l'un de l'autre, tels que décrits dans le présent document. Comme exemple de groupe protecteur alcoxyalcoxyalkyle, on peut citer notamment le méthoxyéthoxyméthyle (-CH₂OCH₂CH₂OCH₃).
« Alkyle » désigne un groupe hydrocarboné aliphatique qui peut être linéaire ou ramifié ayant 1 à 20 atomes de carbone dans la chaîne. Des groupes alkyle préférés ont 1 à 12 atomes de carbone dans la chaîne. Ramifié signifie qu'un ou plusieurs groupes alkyle inférieurs, tels que le méthyle, l'éthyle ou le propyle, sont liés à une chaîne alkyle linéaire. « Alkyle inférieur » signifie de 1 à 4 atomes de carbone dans la chaîne qui peut être linéaire ou ramifiée. Comme exemple de groupe protecteur alkyle en C₃-C₄, on peut citer notamment l'isopropyle (-CH(CH₃)₂) et le t-butye (-C(CH₃)₃).
« Aralkyle » désigne un groupe aryl-alkyl-, dans lequel l'aryle et l'alkyle sont tels que décrits dans le présent document. Des aralkyle préférés contiennent un fragment alkyle inférieur. Des exemples types de groupes protecteurs aralkyle comprennent le benzyle, le groupe -CH₂-anthryl.
« Aroyle » désigne un groupe aryl-CO-, dans lequel le groupe aryle est tel que décrit dans le présent document. Des exemples types de groupes aroyle comprennent le benzoyle et le 1- et 2-naphtoyle.
« Alkylthioalkyle » désigne un groupe alkyl-S-alkyl-, dans lequel le groupe alkyle est tel que décrit dans le présent document. Comme exemple de groupe protecteur alkylthioalkyle, on peut citer notamment le méthylthiométhyle (CH₃SCH₂-).
« Aryle » désigne un système cyclique monocyclique ou multicyclique aromatique ayant de 6 à 14 atomes de carbone, de préférence de 6 à 10 atomes de carbone. Des exemples types de groupes aryle comprennent le phényle ou le naphtyle.
« Arylalcoxyalkyle » désigne un groupe aryl-alkyl-O-alkyl-, dans lequel les groupes aryles et alkyles sont tels que définis dans le présent document. Comme exemple de groupe protecteur arylalcoxyalkyle, on peut citer notamment le benzyloxyméthyle (PhCH₂OCH₂-).
« Cycloalkyle » signifie un système de cycle non aromatique mono- ou multicyclique ayant de 3 à 10 atomes de carbone, de préférence de 5 à 10 atomes de carbone. Les tailles de cycle préférées des cycles du système de cycle comprennent 5 à 6 atomes de cycle. Comme exemple de groupe protecteur cycloalkyle, on peut citer notamment le cyclohexyle *(*cC₆H₁₁).
« Cycloalcoxy » signifie un système de cycle non aromatique mono- ou multicyclique ayant de 2 à 9 atomes de carbone, de préférence de 4 à 9 atomes de carbone et comprenant un atome d'oxygène. Les tailles de cycle préférées des cycles du système de cycle comprennent environ 5 à environ 6 atomes de cycle. Comme exemple de groupe protecteur cycloalcoxy, on peut citer notamment le 2-tétrahydropyranyle.
« Perfluoroaryle » signifie un groupe aryle dans lequel les atomes d'hydrogène sont substitués par des atomes de fluor. Les groupes perfluoroaryle peuvent être substitués par un ou plusieurs groupes perfluoroalkyles inférieurs. Comme exemple de groupe protecteur perfluoroaryle, on peut citer notamment le -C₆F₄CF₃.

De préférence, le groupe aroyle est un groupe benzoyle (Ph-CO-).

### Alkylation énantiosélective

Selon un deuxième objet, l'invention concerne un procédé de préparation d'un énantiomère ou d'un mélange énantiomériquement enrichi d'un composé de formule (I) : 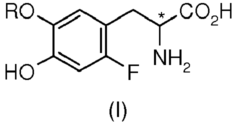 dans laquelle :
R est H ou CH₃ ; et
F est le ¹⁹F ou le ¹⁸F,
comprenant les étapes de :
i) alkylation d'un composé de formule (II) telle que définie ci-dessus par une imine de formule (III-1) : 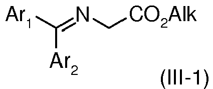 dans laquelle :
   Ar₁, Ar₂ sont des groupes aryles ; et
   Alk représente un groupe alkyle en C₁-C₆ ;
   en milieu biphasique eau/solvant organique, en présence d'un catalyseur de transfert de phase chiral (CTP*) et d'un hydroxyde de métal alcalin ou alcalino-terreux, ce par quoi on obtient un énantiomère ou un mélange énantiomériquement enrichi d'un composé de formule (IV-1) : 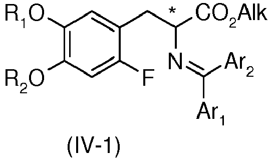
(ii) déprotection des fonctions alcool protégées par un groupement aroyle et déprotection des fonctions amine,acide carboxylique du composé de formule (IV-1), ce par quoi on obtient un énantiomère ou un mélange énantiomériquement enrichi d'un composé de formule (I), éventuellement
(iii) la récupération du composé de formule (I) obtenu.

Par « mélange énantiomériquement enrichi», on entend un mélange comprenant un excès énantiomérique supérieur à 50 %, de préférence supérieur à 80 % et notamment supérieur à 90%.

De préférence, le composé de formule (I) est un énantiomère ou un mélange énantiomériquement enrichi de la 6-fluoro-Dopa, i.e. R=H.

De préférence, F est un atome de fluor ¹⁸F.

Selon un aspect particulièrement préféré, le composé de formule (I) est le composé 6-F-L-Dopa de formule :

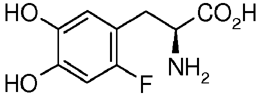

De préférence, le groupe GP est un groupe aroyle notamment un groupe Ph-CO-.

De façon surprenante, il a été montré que les groupements protecteurs GP tels que définis ci-dessus, dont notamment le groupe benzoyle, bien que plus labiles que les groupes méthyles décrits dans l'art antérieur, résistent bien aux conditions basiques en milieu aqueux mises en oeuvre dans ce procédé, permettant ainsi d'obtenir le composé (IV-1) avec de bons rendements.

L'hydroxyde de métal alcalin ou alcalino-terreux peut être notamment l'hydroxyde de sodium, l'hydroxyde de potassium, ce dernier étant particulièrement préféré. Ces hydroxydes sont en effet moins onéreux que l'hydroxyde de césium mis en oeuvre dans le procédé énantiosélectif décrit dans l'art antérieur. De plus, contrairement à l'hydroxyde de césium, ils sont solubles dans l'eau et permettent d'obtenir une meilleure reproductibilité de la réaction.

Le catalyseur de transfert de phase chiral CTP* est un catalyseur qui comprend au moins un centre chiral et qui possède une configuration absolue. Le CTP* exerce une rotation (+) ou (-) du plan de polarisation d'une lumière polarisée.

Le CTP* peut être un sel d'ammonium quaternaire, notamment choisi parmi:
- un composé de formule (A) : 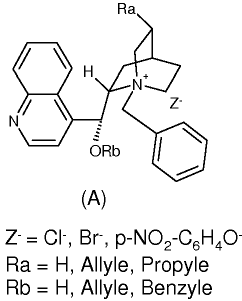
- un composé de formule (B) : 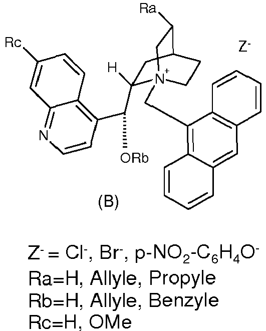
- un composé de formule (C) : 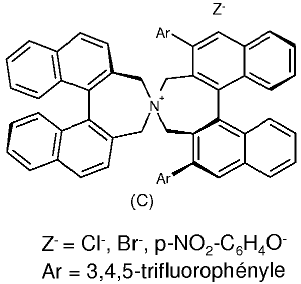
- un composé de formule (D) : 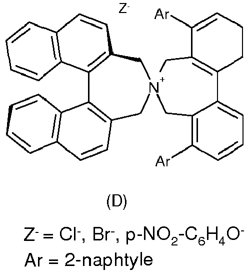 ou
- un composé de formule (E) :

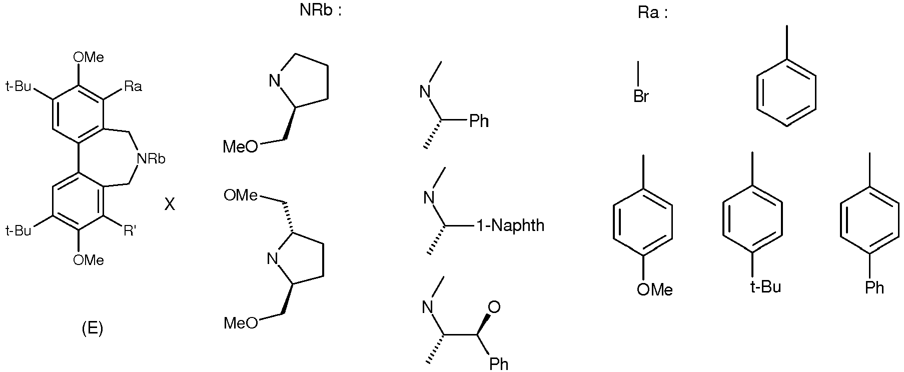

Sans vouloir se limiter à une théorie particulière, il a été montré que le sel d'ammonium quaternaire n'était pas en mesure de transporter l'ion hydroxyde vers la phase organique comme dans un cas classique de transfert de phase. La déprotonation de l'imine (III-1) se produit à l'interphase. L'énolate et son contre-ion ammonium résultant diffusent ensuite vers la phase organique où a lieu la réaction d'alkylation énantiosélective.

L'énantiosélectivité de cette alkylation s'explique par le fait que l'une des faces de l'énolate est bloquée par le contre-ion, permettant ainsi la stéréodifférenciation. L'attaque du composé de formule (II) se déroule donc sur la face libre de l'énolate.

De préférence, le CTP* est le composé de formule (B1) : 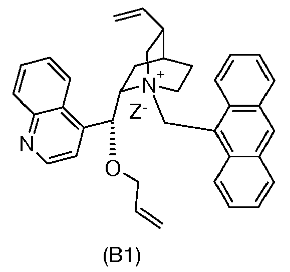 dans laquelle Z est choisi parmi Cl, Br, ou I.

De préférence, le composé de formule (III-1) est le composé de formule (III-1a) :

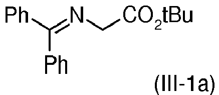

Il n'y a pas de restriction particulière sur la nature du solvant organique à employer, à condition qu'il n'ait aucun effet indésirable sur la réaction ou sur les réactifs impliqués. Les exemples de solvants adaptés comprennent notamment les hydrocarbures aromatiques, tels que le toluène.

### Alkylation diastéréosélective

Selon un autre objet, l'invention concerne un procédé de préparation d'un énantiomère ou d'un mélange énantiomériquement enrichi d'un composé de formule (I) tel que défini ci-dessus, comprenant :
(i) l'alkylation d'un composé de formule (II) tel que défini ci-dessus, par une imine de formule (III-2) : 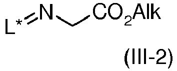 dans laquelle L* représente une copule chirale, et Alk un groupe alkyle en C₁-C₆, en présence d'une base dans un solvant organique, ce par quoi on obtient un diastéréoisomère ou un mélange diastéréoisomériquement enrichi de formule (IV-2) : 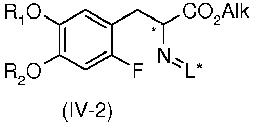
(ii) la déprotection des fonctions alcool protégées par un groupement aroyle et déprotection des fonctions amine, acide carboxylique du composé de formule (IV-2), ce par quoi on obtient un énantiomère ou un mélange énantiomériquement enrichi d'un composé de formule (I), et éventuellement;
(iii) la récupération du composé de formule (I) obtenu.

La copule chirale L* est un groupement chimique comprenant au moins un centre chiral, notamment un carbone asymétrique. Le ligand chiral exerce une rotation (+) ou (-) du plan de polarisation d'une lumière polarisée.

De préférence, l'imine de formule (III-2) est le composé de formule (III-2a) :

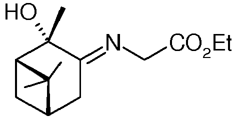

La base, généralement soluble dans le solvant organique, est capable de déprotoner l'un des atomes d'hydrogène en α de la fonction ester de l'amine (III-2). De préférence, il s'agit d'une base organique, telle qu'une amine. Comme exemples de bases particulièrement préférées, on peut citer en particulier les sels de métal alcalin ou alcalino-terreux d'une amine primaire ou secondaire, notamment les sels de métal alcalin ou alcalino-terreux d'alkylamine en C₁-C₁₀, ou de bistrialkylsilylamine en (C₁-C₆), tel que le buthyllithium ou le lithium hexaméthyldisilazane (LHMDS).

De préférence, la quantité de base est en excès par rapport à l'imine de formule (III-2) et représente notamment 2 équivalents molaires par rapport à l'imine (III-2).

Il n'y a pas de restriction particulière sur la nature du solvant organique à employer, à condition qu'il n'ait aucun effet indésirable sur la réaction ou sur les réactifs impliqués. Les exemples de solvants préférés comprennent notamment les solvants aprotiques, notamment polaires, tels que l'éther diéthylique ou le tétrahydrofuranne.

Selon une variante particulièrement préférée du procédé diastéréosélectif selon l'invention, l'étape i) est réalisée en présence de l'imine de formule (III-2a), de LHMDS, dans l'éther, selon le schéma de synthèse suivant :

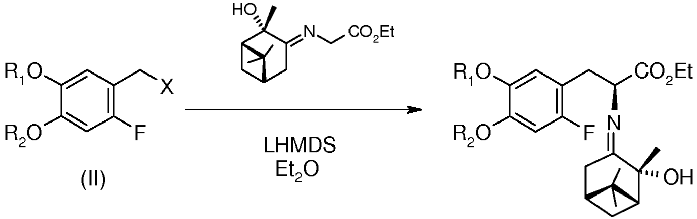

De façon avantageuse, ces conditions permettent d'améliorer la diastéréosélectivité de la réaction par rapport au procédé de Lemaire et al., 1991 (voir schéma 1 ci-dessus). Ainsi, dans ces conditions, la diastéréosélectivité est supérieure à 99% et l'imine de formule (III-2a) est obtenue avec un rendement supérieur à 80%. En outre, aucune racémisation de l'imine obtenue n'est observée lors de l'étape subséquente ii) de déprotection, laquelle est généralement réalisée dans des conditions relativement douces.

Selon un mode de réalisation préféré, l'étape ii) de déprotection comprend une hydrolyse acide du composé de formule (IV-1) ou (IV-2) en présence d'un antioxydant, ce par quoi on obtient un énantiomère ou un mélange énantiomériquement enrichi d'un composé de formule (I).

Par "hydrolyse acide", on entend une hydrolyse réalisée dans des conditions de pH inférieur à 2 notamment inférieur à 1.

De préférence, l'hydrolyse acide est réalisée en chauffant le milieu réactionnel à reflux, i.e à une température proche de la température d'ébullition de l'eau (environ 100°C).

L'étape de déprotection ii) est réalisée en présence d'un antioxydant de manière à éviter l'oxydation en hydroquinone du catéchol obtenu après déprotection des fonctions alcools. L'antioxydant est de préférence soluble dans l'eau. Comme exemple d'antioxydant utile selon le procédé de l'invention on peut notamment citer le phénol ou l'acide ascorbique.

L'étape de déprotection (ii) peut également être réalisée en deux étapes successives :
ii1) déprotection de la fonction amine avec une solution acide peu concentrée, de pH compris entre 3 et 4 environ, par exemple une solution d'acide citrique à 15% en poids dans l'eau ;
ii2) déprotection de la ou des fonctions alcool protégées par un groupe aroyle et de la fonction acide carboxylique protégée selon une hydrolyse acide en présence d'un antioxydant, telle que décrite ci-dessus.

Cette déprotection en deux étapes peut être utile pour mesurer l'énantiosélectivité de l'alkylation (étape i) à l'issue de l'étape ii1).

Les composés de formule (II) et les intermédiaires (VI) et (VII) décrits ci-après peuvent être préparés par l'application ou l'adaptation de procédés connus, par exemple les procédés tels que décrits dans les Exemples ou leurs équivalents chimiques évidents.

Selon une variante particulière de l'invention, le composé de formule (II) est préparé à partir d'un composé de formule (VI) : 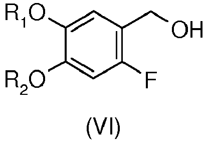 dans laquelle R₁, R₂, F sont tels que définis ci-dessus selon les méthodes classiques d'halogénation des alcools. A titre d'exemple, les composés de formule (II) peuvent être préparés selon un procédé comprenant la réaction du composé de formule (VI) avec PPh₃, I₂ en présence d'imidazole dans le dichlorométhane.

Le composé de formule (VI) peut être préparé à partir d'un composé de formule (VII) : 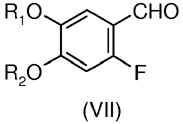 dans laquelle R₁, R₂, F sont tels que définis ci-dessus selon les méthodes classiques de réduction des aldéhydes. A titre d'exemple, les composés de formule (VI) sont préparés selon un procédé comprenant la réaction du composé de formule (VII) avec un hydrure métallique, en présence d'eau dans un solvant polaire aprotique.

Le composé (VII) peut être préparé à partir du 6-fluorovératraldéhyde de formule (VIII) : 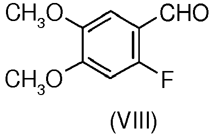 dans laquelle F est tel que défini ci-dessus, notamment selon un procédé comprenant la réaction du composé de formule (VIII) avec un composé R₁Y ou R₂Y où Y représente Br, I, Cl et R₁, R₂ sont tels que définis ci-dessus, en présence d'hexabutylguanidinium.HCl (HBGC.HCl) à titre de catalyseur dans un solvant aromatique. Pour plus d'informations concernant cette étape de transprotection, on pourra se référer à la publication C. Marette et al., 2006.

Le composé de formule (VIII) où F est le ¹⁹F est disponible commercialement.

Le composé de formule (VIII) où F est le ¹⁸F peut être préparé à partir du 6-nitrovératraldéhyde, disponible commercialement, selon une méthode classique de substitution nucléophile du groupe NO₂ par un anion ¹⁸F (C. Lemaire et al., 1989 et 1991, page 1, lignes 24-25). A titre d'exemple, le composé de formule (VIII) marqué au ¹⁸F peut être préparé par réaction du 6-nitrovératraldéhyde avec un complexe KF+[18]C6 dans le DMSO.

Selon un autre objet, l'invention concerne les composés de formule (VI) : 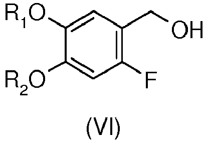 dans laquelle F, R₁ et R₂ sont tels que définis ci-dessus.

L'invention a également pour objet les composés de formule (VII) : 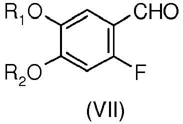 dans laquelle F, R₁ et R₂ sont tels que définis ci-dessus, à l'exclusion du composé dans lequel R₁=R₂= Ph(CO)- et F est ¹⁹F.

Les composés (VI) et (VII) sont particulièrement utiles pour la préparation de composés de formule (II) selon l'invention.

### EXEMPLES

### Matériels et méthodes

Les spectres RMN ¹H ont été réalisés sur des spectromètres Brücker AC-250, Avance-300, ARX-400 et Avance-500 avec cryosonde. Les déplacements chimiques (δ) sont exprimés en parties par million (ppm) par rapport au tétraméthylsilane (TMS). Les multiplicités sont données comme suit : s pour singulet, d pour doublet, t pour triplet, q pour quadruplet et m pour massif. Les constantes de couplage J sont exprimées en Hertz.

Les spectres ¹³C ont été réalisés en mode J modulé sur des spectromètres Brücker AC-250, Avance-300 WB, ARX-400 WB et Avance-500 avec cryosonde. La référence est également le TMS.

Les spectres ¹⁹F découplés ¹H ont été réalisés sur un spectromètre Brücker AC-200 (référence : CF₃CO₂H) ainsi que sur des spectromètres Avance-300 WB et Brücker ARX-400 (référence : CFCl₃).

Les spectromètres de masse ont été obtenus grâce à :
- un spectromètre Nermag R10-10H en mode d'ionisation chimique (DCI : Desorption Chemical Ionisation), en mode d'impact électronique (DEI : Desorption Electronic Impact), en mode bombardement atomique rapide (FAB : Fast Atomic Bombardement),
- un spectromètre Perkin Elmer SCIEX API 365 ou un spectromètre Q-TRAP Applied Biosystems en mode électrospray (ES ou ion spray IS).

Les spectres infrarouges ont été réalisés entre lames de NaCl ou à l'aide de pastilles de KBr sur un spectromètre Perkin Elmer FI/IR 1725X. Les nombres d'ondes ν des principales fonctions sont indiqués en cm⁻¹.

Les points de fusion ont été mesurés sur un appareil Büchi à capillaire.

Les analyses élémentaires ont été réalisées au service de micro-analyse du Laboratoire de Chimie de Coordination de Toulouse.

La pureté des produits ainsi que l'avancement des réactions ont été contrôlés par CCM sur plaques de gel de silice Alugram sil G/UV254nm d'épaisseur 0.2 mm (Macherey-Nagel).

La plupart des purifications ont eu lieu sur MPLC (chromatographie liquide moyenne pression) miniprep Jobin Yvon avec des colonnes de 20 ou 40 mm de diamètre (silice 60 Merck 15 - 40 µm).

Les chromatographies analytiques ont été effectuées sur un chromatographe Waters (Pompe 600, Injecteur 717 plus et UV barette de diode 996).

La détermination cristallographique a été réalisée sur un appareil Brücker AXS CCD1000.

L'appareil micro-ondes mono-mode est de marque CEM. Il fonctionne à une fréquence de 2450 MHz sous contrôle de température, en réacteur ouvert ou fermé.

Dans les exemples ci-après, l'abréviation "éq." se réfère à des équivalents en nombre de moles.

### Méthode générale de réduction des aldéhydes

A 1,0 éq. de réactif en solution dans du tétrahydrofuranne (THF) est ajoutée lentement une solution aqueuse de NaBH₄ (2,0-3,0 éq.). Après 15 minutes d'agitation à température ambiante (TA), le milieu réactionnel est extrait avec de l'AcOEt. La phase organique est lavée avec une solution saturée en NaHCO₃ puis avec une solution saturée en NaCl. Elle est ensuite séchée sur MgSO₄ et évaporée sous vide.

### Méthode générale d'iodation des alcools

A 1,5-1,7 éq. de triphénylphosphine en solution dans du dichlorométhane (DCM) à 0°C sont ajoutés lentement 1,5-1,7 éq. de I₂. Le mélange est maintenu sous agitation pendant 10 minutes, puis une solution de DCM contenant 1,0 éq. d'alcool ainsi que 3,0-3,5 éq. d'imidazole est ajoutée au milieu réactionnel. Après 15 minutes d'agitation à 0°C, le mélange est lavé avec une solution de NaHSO₃ 5%. La phase organique est évaporée puis filtrée sur une petite colonne de silice (éluant : DCM). Le filtrat est séché sur MgSO₄, filtré puis évaporé sous vide. Le composé est conservé au congélateur sous atmosphère inerte.

### Méthode générale d'alkylation énantiosélective

### 1^{re} étape :

A 0,2 éq. de bromure de 9-(allyloxy)-1-(9-anthrylméthyl)cinchonan-1-ium 13 en solution dans du toluène sont ajoutés 1,2 éq. d'ester de t-butyl N-(diphénylméthylène)glycine ainsi qu'une solution aqueuse de KOH 50% en large excès. Le mélange est agité pendant 30 minutes à TA puis 1,0 éq. de réactif iodé en solution dans du toluène est ajouté lentement. L'avancement de la réaction est suivi par RMN ¹H. Après consommation du dérivé iodé, les phases sont séparées et le toluène est évaporé. Du DCM est ensuite ajouté. La solution est passée sur une petite cartouche de MgSO₄ puis évaporée.

### 2^{e} étape :

Le résidu obtenu lors de la première étape est solubilisé dans du THF puis de l'acide citrique 15% aq. est ajouté. Le milieu réactionnel est maintenu sous agitation à TA pendant 2 heures et est extrait à l'Et₂O. La phase aqueuse est neutralisée à l'aide d'une solution saturée en NaHCO₃ puis extraite au DCM. La phase organique est lavée avec une solution saturée en NaCl, séchée sur MgSO₄ puis évaporée.

### Méthode générale de transprotection

A 2 mmoles de dérivé méthoxyarylé sont ajoutés 0,2 éq. de HBGC.HCl (séchés préalablement à vide) ainsi que 1,25 éq. de chlorure de benzoyle. Suivant la solubilité de l'aryle dans le chlorure de benzoyle, la réaction a lieu, ou non, en présence d'o-dichlorobenzène (o-DCB). Le milieu réactionnel est agité sous champ micro-ondes à 180°C. Après refroidissement, le milieu est dilué au DCM, filtré sur gel de silice et concentré sous pression réduite. La solution concentrée est ajoutée à 50 ml d'EP d'éther de pétrole. Le précipité obtenu est filtré et séché sous pression réduite.

### Exemple 1 :

### 6-nitrovératraldéhyde ou 4,5-diméthoxy-2-nitrobenzaldéhyde

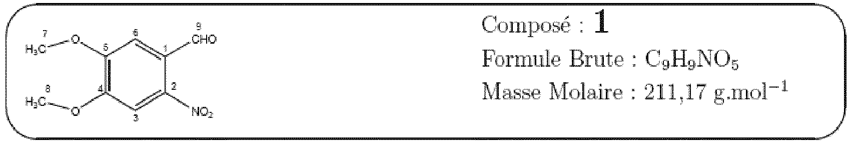

Produit commercial : Alfa Aesar
RMN ¹H (CDCl₃, 400 MHz) : 4,01 (3H, s, CH₃(7)) ; 4,02 (3H, s, CH₃(8)) ; 7,38 (1 H, s,H(6)) ; 7,59 (1 H, s, H(3)) ; 10,39 (1 H, s, CHO).
RMN ¹³C (CDCl₃, 100 MHz) : 56,94 (OCH₃) ; 57,01 (OCH₃) ; 107,4 (C3) ; 109,9 (C6) ; 125,7 (C1) ; 144,1 (C2) ; 152,6 (C4) ; 153,4 (C5) ; 187,9 (C9).
IR (KBr, v) : 1685 (C=O) ; 1523 (NO₂ asym) ; 1338 (NO₂ sym) ; 1227 (Csp2-O) ; 1063 (Csp3 -O) ; 878 (C-N).
Rf : 0,49 (DCM : 100%).

### HPLC :

- Chromlith SpROD RP 18e 50x4,6
- Eau/ACN : 80/20 gradient (t=0,5 min : 80/20 → t=3,5 min : 20/80)
- Débit : 2 ml/min
- tR=2,9 min
- UV : 263 nm; 310 nm; 350 nm.

### Exemple 2 :

### 6-fluorovératraldéhyde ou 2-fluoro-4,5-diméthoxybenzaldéhyde

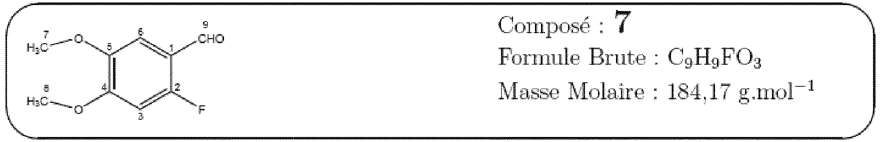

### Méthode A :

### Synthèse de [18]C6-KF 8 :

Selon la procédure décrite par Bourrel et al. (J. Phys. Org. Chem., 2001), à 139 mg (2,39 mmol ; 1,0 éq.) de KF sont additionnés 643 mg (2,43 mmol ; 1,0 éq.) de 1,4,7,10,13,16-hexaoxacyclooctadécane ([18]C6) puis 5 ml d'eau distillée. Un appareil de Dean-Stark est adapté puis le milieu réactionnel est chauffé à reflux pendant 10 minutes. 5 ml d'ACN sont ensuite ajoutés à la solution afin de former l'azéotrope et d'éliminer ainsi l'eau du milieu. L'opération est réitérée quatre fois, puis le mélange est évaporé sous pression réduite et lyophilisé. Le complexe KF + [18]C6 **8** est obtenu sous la forme d'un solide blanc.

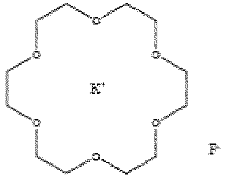

### Fluoration :

772 mg (2,40 mmol ; 2,0 éq.) de complexe KF + [18]C6 **8** sont ajoutés à 250 mg (1,18 mmol ; 1,0 éq.) de nitrovératraldéhyde **1** en solution dans 10 ml de DMSO. Après 24 heures d'agitation à 150°C, le milieu réactionnel est extrait à l'Et₂O. La phase organique est lavée avec de l'eau, une solution saturée en NaHCO₃ puis avec une solution saturée en NaCl. Cette phase est ensuite séchée sur MgSO₄ puis évaporée sous pression réduite. 53 mg de brut sont purifiés sur une colonne ouverte (silice ; DCM/EP : 70/30). 9 mg (4%) de 6-fluorovératraldéhyde **7** sont obtenus sous la forme d'un solide blanc.

### Méthode B :

A 10,50 g (49,74 mmol, 1,0 éq.) de nitrovératraldéhyde **1** en solution dans 250 ml de MeOH sont lentement additionnés 3,87 g (3,64 mmol ; 0,1 éq.) de Pd/C à 10%. La solution est dégazée à l'argon pendant 20 minutes puis le flux d'argon est remplacé par un flux d'hydrogène. Après 5 heures d'agitation à température ambiante sous H₂, le mélange est filtré sur célite puis évaporé sous pression réduite. Le brut est repris dans 40 ml de MeOH et agité à -5°C. 4,57 g (66,28 mmol ; 1,3 éq.) de NaNO₂ sont lentement additionnés au milieu réactionnel puis 42,5 ml (0,31 mol ; 6,2 éq.) d'HBF₄ à 54 % dans l'Et₂O. Après 1 heure d'agitation à -5°C, le mélange est versé dans 200 ml d'Et₂O froid. Le mélange est filtré puis lavé plusieurs fois à l'Et₂O froid. 12,72 g (91 %) de tétrafluoroborate de 2-formyl-4,5-diméthoxybenzènediazonium **9** sont obtenus sous la forme d'un solide marron. 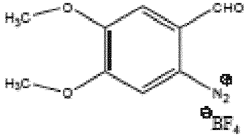 IR composé **9** (KBr, ν) : 2238 (NN) ; 1639 (C=O) ; 1298 (Cₛₚ₂-O) ; 1052 (Cₛₚ₃-O).

2,06 g (7,36 mmol ; 1,0 éq.) de tétrafluoroborate de 2-formyl-4,5-diméthoxy-benzènediazonium 9 sont placés dans un ballon équipé d'un piège à eau. Le ballon est chauffé (~ 300-350°C). Le chauffage est maintenu pendant le dégagement de vapeurs brunes. Après retour à TA, le mélange est extrait à l'AcOEt. La phase organique est lavée avec une solution saturée en NaHCO₃ puis avec une solution saturée en NaCl, elle est ensuite séchée sur MgSO₄ puis évaporée sous pression réduite. 418 mg de brut sont purifiés sur MPLC (silice ; DCM). 232 mg (16%/1) de 6-fluorovératraldéhyde **7** sont obtenus sous la forme d'un solide blanc.
RMN ¹H (CDCl₃, 400 MHz) : 3,87 (3H, s, CH₃(7)) ; 3,92 (3H, s, CH₃(8)) ; 6,62 (1 H, d, J=11,6 Hz, H(3)) ; 7,23 (1 H, d, J=6,4 Hz, H(6)) ; 10,18 (1 H, s, CHO).
RMN ¹³C (CDCl₃, 75 MHz) : 56,5 (C7) ; 56,7 (C8) ; 99,7 (d, J=26,9 Hz, C3) ; 107,8 (d, J=3.6 Hz, C6) ; 116,4 (d, J=8,6 Hz, C1) ; 146,2 (C5) ; 156,0 (d, J=10,7 Hz, C4) ; 161,4 (d,J=253,6 Hz, C2) ; 185,9 (d, J=6,5 Hz, C9).
RMN ¹⁹F (CDCl₃, 188 MHz) : -52,67 ppm.
RMN ¹⁹F (CDCl₃, 376 MHz) : -128,30 ppm.
SM (DCl/NH₃, m/z) : 185 [M+H⁺] ; 202 [M+NH₄⁺] ; 219 [M+N₂H₇⁺].
IR (KBr, ν) : 1661 (C=O) ; 1276 (Cₛₚ₂-O) ; 1219 (Ar-F) ; 1112 (Cₛₚ₃-O).
Pf : 106°C (commercial : 93-97°C Aldrich).
Rf : 0,22 (DCM : 100%).

### HPLC:

- Nucleosil 100-3 CC70-3 MN C18 HD 5 µm
- tampon (AcOH/AcONH+4 pH=3,8)/ACN : 75/25 isocratique
- Débit : 0,6 ml/min
- tR=4,3 min
- UV : 228 nm; 272 nm; 317 nm.

### HPLC:

- Chromlith SpROD RP 18e 50x4,6
- Eau/ACN : 80/20 gradient (t=1 min : 80/20 → t=7 min : 20/80)
- Débit : 2 ml/min
- tR=2,6 min.

### Exemple 3 :

### Chlorure de 1-(9-anthrylméthyl)cinchonan-1-ium-9-ol

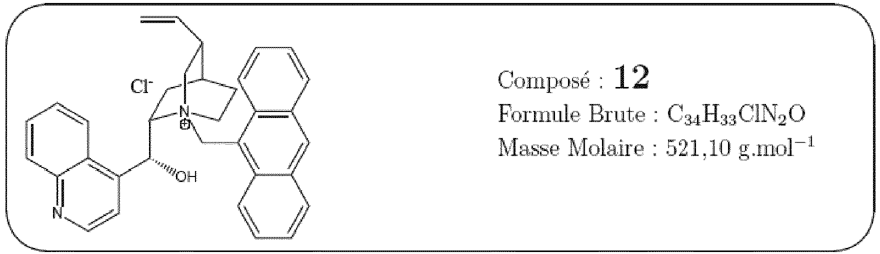

Selon la procédure décrite par Corey et al. (J. Am. Chem. Soc., 1997), à 6,11 g (20,77 mmol ; 1,0 éq.) de cinchonidine en solution dans 100 ml de toluène sont additionnés 4,71 g (20,77 mmol ; 1,0 éq.) de 9-(chlorométhyl)anthracène. Après 3 heures d'agitation à reflux, le mélange réactionnel est ramené à température ambiante, puis versé dans 200 ml d'Et₂O. Le solide ainsi formé est lavé plusieurs fois à l'Et₂O (4 fois 50 ml) puis est évaporé sous pression réduite. 10,25 g (95%) de chlorure de 1-(9-anthrylméthyl)cinchonan-1-ium-9-ol 12 sont obtenus sous la forme d'une poudre jaune clair.
RMN ¹H (CDCl₃, 300 MHz) : 9,07 (1 H, d, J=8,7 Hz) ; 8,86 (2H, m) ; 8,75 (1 H, d, J=9,0 Hz) ; 8,21 (1 H, d, J=5,2 Hz) ; 8,04 (1 H, d, J=4,5 Hz) ; 8,00 (1 H, s) ; 7,58-7,74 (3H, m) ; 7,39-7,44 (1 H, m) ; 7,07-7,30 (6H, m) ; 6,85 (1 H, d, J=12 Hz) ; 6,69 (1 H, d, J=12 Hz) ; 5,39-5,50 (1 H, m) ; 5,25 (1 H, d, J=17,7 Hz) ; 4,90-5,01 (1 H, m) ; 4,69-4,78 (2H, m) ; 4,05-4,09 (1 H, m) ; 2,60 (1 H, dd, J=12,8 Hz, J'=10,5 Hz) ; 2,43 (1 H, t, J=11,5 Hz) ; 2,1-2,15 (1H, m) ; 1,77-1,88 (2H, m) ; 1,70 (1H, s) ; 0,98-1,2 (2H, m).
RMN ¹³C (CDCl₃, 75 MHz) : 23,3 ; 25,7 ; 25,9 ; 38,5 ; 50,6 ; 55,0 ; 61,3 ; 67,1 ; 67,3 ; 117,7 ; 118,2 ; 120,1 ; 124,0 ; 124,2 ; 124,8 ; 124,8 ; 125,7 ; 126,1 ; 126,8 ;
127,4 ; 127,7 ; 128,3 ; 128,5 ; 128,6 ; 129,3 ; 130,3 ; 130,4 ; 131,1 ; 132,7 ; 133,2 ; 136,4 ; 145,7 ; 147,2 ; 149,4.
SM (ES+/CH₃OH, m/z) : 485,35 ([M-Cl]⁺).
Pf : 160-162°C (Lit. 168°C, Corey et al., 1997).

### Exemple 4 :

### Bromure de 9-(allyloxy)-1-(9-anthrylméthyl)cinchonan-1-ium

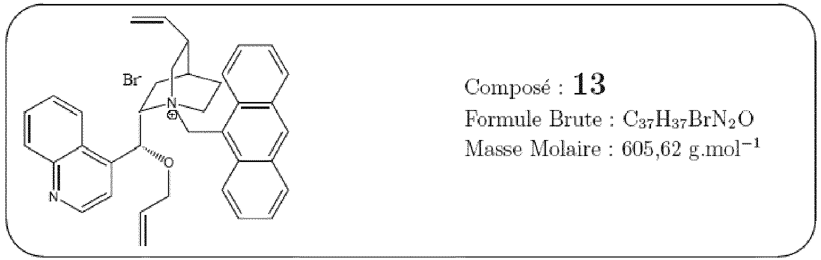

Selon la procédure décrite par Corey et al. (J. Am. Chem. Soc., 1997), à 10,08 g (19,34 mmol ; 1,0 éq.) de chlorure de N-9-anthracèneméthylcinchonidine 12 obtenu précédemment en solution dans 100 ml de DCM anhydre sont lentement additionnés 5,3 ml (61,24 mmol ; 3,2 éq.) de bromure d'allyle ainsi que 10,5 ml (93,57 mmol ; 4,8 éq.) de KOH 50%. Après 4 heures d'agitation à température ambiante, 100 ml d'eau distillée sont ajoutés au mélange réactionnel. La phase organique est lavée avec une solution saturée en NaHCO₃ puis avec une solution saturée en NaCl, elle est ensuite séchée sur MgSO₄, filtrée puis évaporée sous pression réduite. Le solide orange obtenu est recristallisé dans un mélange DCM/Et₂O. 8,67 g (74%) de bromure de 9-(allyloxy)-1-(9-anthryl-méthyl)cinchonan-1-ium **13** sont obtenus sous la forme d'une poudre orange. RMN ¹H (CD₃OD, 300 MHz) : 1,37-1,55 (2H, m) ; 1,84-1,87 (1H, m) ; 2,04-2,11 (1 H,m) ; 2,33-2,43 (2H, m) ; 2,75-2,85 (1 H, m) ; 3,14 (1 H, t, J=12,3 Hz) ; 3,65-RMN 1 H (CD₃OD, 300 MHz) : 1,37-1,55 (2H, m) ; 1,84-1,87 (1 H, m) ; 2,04-2,11 (1 H, m) ; 2,33-2,43 (2H, m) ; 2,75-2,85 (1 H, m) ; 3,14 (1 H, t, J=12,3 Hz) ; 3,65-3,74 (1 H, m) ; 4,29-4,47 (4H, m) ; 4,86-4,92 (2H, m) ; 5,45 (1 H, dd, J=10,6 Hz, J'=1,3 Hz) ; 5,53-5,65 (2H, m) ; 5,80 (1 H, d, J=14,1 Hz) ; 6,24-6,37 (2H, m) ; 6,85 (1 H, s) ; 7,51-7,57 (2H, m) ; 7,63-7,73 (2H, m) ; 7,80-7,88 (3H, m) ; 8,09-8,16 (3H, m) ; 8,35 (1 H, d, J=9 Hz) ; 8,47-8,49 (1 H, m) ; 8,66 (1 H, d, J=9,2 Hz) ; 8,79 (1 H, s) ; 8,94 (1 H, d, J=4,6 Hz).
RMN ¹³C (CD₃OD, 100 MHz) : 21,9 ; 24,8 ; 25,9 ; 38,1 ; 52,3 ; 56,0, 62,1 ; 68,7 ; 70,0 ; 116,4 ; 117,5 ; 117,7 ; 120,3 ; 123,0 ; 123,5 ; 124,0 ; 125,2 ; 125,3 ; 125,7 ; 127,9 ; 128,0 (2C) ; 129,2 ; 129,8 ; 129,9 ; 130,1 ; 131,6 ; 131,7 ; 132,5 ; 133,2 ; 133,3 ; 133,4 ; 137,1 (2C) ; 141,6 ; 148,0 ; 149,7.

**Analyse élémentaire :**

| | |
|---|---|
| Calculée pour C₃₇H₃₇BrN₂O : | C, 73,38 ; H, 6,16 ; N, 4,62 |
| Mesurée : | C, 71,43 ; H, 5,63 ; N, 4,34. |
| (Commercial : | C, 71,54 ; H, 5,98 ; N, 4,42) |

SM (ES+/CH₃OH, m/z) : 525,3 ([M-Br]⁺).
Pf : 172-175°C (Lit. 194-196°C, Corey et al 1997).

### Exemple 5 :

### Dibenzoate de 4-fluoro-5-formyl-1,2-phénylène

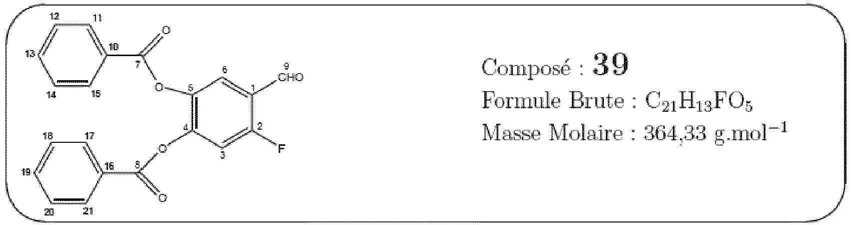

### Méthode A :

D'après la procédure décrite par Olofson Roy A. et al (1994, Brevet N°US5283358), à 111 mg (0,23 mmol ; 0,2 éq.) de chlorhydrate de chlorure d'hexabutylguanidinium (HBGC.HCl), préalablement séchés pendant 30 minutes sous pression réduite et en solution dans 5 ml de 1,2-dichlorobenzène sont additionnés 202 mg (1,09 mmol ; 1,0 éq.) de 6-fluorovératraldéhyde 7 puis 315 µl (2,71 mmol ; 2,5 éq.) de chlorure de benzoyle. Après 8 heures de chauffage à reflux, le 1,2-dichlorobenzène est évaporé (80°C, 1 mmHg). Le brut est purifié sur une colonne MPLC (silice ; EP/DCM/AcOEt : 90/8/2). 335 mg (84%) de dibenzoate de 4-fluoro-5-formyl-1,2-phénylène **39** sont obtenus sous la forme d'un solide blanc.

### Méthode B :

Même schéma que la méthode A mais par irradiation micro-ondes en pression atmosphérique avec les quantités suivantes :
6,01 g (32,65 mmol ; 1,0 éq.) de 6-fluorovératraldéhyde 7
3,15 g (6,73 mmol ; 0,2 éq.) de HBGC-HCl
9,5 ml (81,84 mmol ; 2,5 éq.) de chlorure de benzoyle
9,5 ml de o-dichlorobenzène.
Le milieu réactionnel est chauffé à 180°C pendant 90 minutes à l'aide des micro-ondes. Après retour à TA, environ 10 g de silice sont rajoutés à la solution bleutée ainsi que 50 ml de DCM. Le tout est mis sous agitation à TA pendant 1 heure. La solution est ensuite filtrée puis le DCM est quasi-évaporé. 150 ml d'EP sont rajoutés pour précipiter le produit. 10,10 g (85%) de dibenzoate de 4-fluoro-5-formyl-1,2-phénylène **39** sont obtenus sous la forme d'un solide blanc.
RMN ¹H (CDCl₃, 400 MHz) : 7,36-7,44 (4H, m, H(12), H(14), H(18), H(20)) ; 7,41 (1 H, d, J=10,2 Hz, H(3)) ; 7,55-7,61 (2H, m, H(13), H(19)) ; 7,91 (1 H, d, J=6,7 Hz, H(6)) ; 8,02-8,09 (4H, m, H(11), H(15), H(17), H(21)) ; 10,34 (1 H, s, H(9)).
RMN ¹³C (CDCl₃, 100 MHz) : 112,7 (d, J=25,6 Hz, C3) ; 122,3 (d, J=9,9 Hz, C1) ; 123,0 (C6) ; 128,1 (C10) ; 128,3 (C16) ; 128,9 (C12, C14, C18, C20) ; 130,5 (C11, C15, C17, C21) ; 134,3 (C19) ; 134,5 (C13) ; 139,8 (d, J=3,4 Hz, C5) ; 148,7 (d, J=12,0 Hz, C4) ; 162,2 (d, J=258,7 Hz, C2) ; 163,4 (C=O-O) ; 164,2 (C=O-O) ; 185,5 (d, J=5,8 Hz, C9).
RMN ¹⁹F (CDCl₃, 188 MHz) : -46,37 ppm.

**Analyse élémentaire :**

| | |
|---|---|
| Calculée pour C₂₁H₁₃FO₅ : | C, 69,23 ; H, 3,60. |
| Mesurée : | C, 68,87 ; H, 3,05. |

SM (DCl/NH₃, m/z) : 382 [M+NH₄⁺].
IR (KBr, v) : 1752 (C=O ester) ; 1697 (C=O aldéhyde) ; 1259 (C-O ester) ; 1230 (Ar-F) ; 1107 (C-O ester).
Pf : 115°C.
Rf : 0,15 (DCM/EP : 50/50).

### HPLC:

- Chromlith SpROD RP 18e 50x4,6
- Eau/ACN : 50/50 isocratique
- Débit : 2 ml/min
- tR=2,8 min
- UV : 242 nm; 354 nm; 368 nm.

### HPLC:

- Chromlith SpROD RP 18e 50x4,6
- Eau/ACN : 80/20 gradient (t=1 min : 80/20 → t=7 min : 20/80)
- Débit : 2 ml/min
- tR=4,3 min.

### Exemple 6 :

### Dibenzoate de 4-fluoro-5-(hydroxyméthyl)-1,2-phénylène

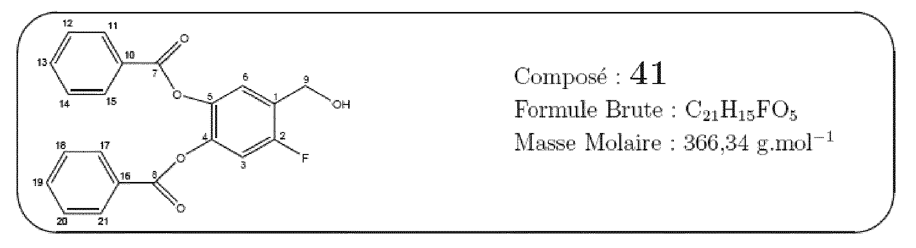

Méthode générale de réduction des aldéhydes avec les quantités suivantes : Dibenzoate de 4-fluoro-5-formyl-1,2-phénylène **39** : 106 mg (0,29 mmol ; 1,0 éq.) dans 5 ml de THF.
NaBH₄ : 22 mg (0,59 mmol ; 2,0 éq.) dans 5 ml d'eau.
Rendement : 78% (MPLC (silice ; DCM/Et₂O : 97/3)).
Aspect : solide blanc.
RMN ¹H (CDCl₃, 400 MHz) : 4,80 (2H, s, CH₂(9)) ; 7,19 (1 H, d, J=9,7 Hz, H(3)) ; 7,36-7,41 (4H, m, H(12), H(14), H(18), H(20)) ; 7,51 (1 H, d, J=7 Hz, H(6)) ; 7,54-7,58 (2H, m, H(13) ; H(19)) ; 8,04-8,08 (4H, m, H(11), H(15), H(17), H(21)).
RMN ¹³C (CDCl₃, 100 MHz) : 58,7 (d, J=3,7 Hz, C9) ; 111,4 (d, J=25,8 Hz, C3) ; 123,4 (d, J=5,9 Hz, C6) ; 127,0 (d, J=16,7 Hz, C1) ; 128,6 (C10, C16) ; 128,8 (C12, C14, C18, C20) ; 130,4 (C11, C15, C17, C21) ; 134,1 (C13, C19) ; 138,9 (d, J=3,6 Hz, C5) ; 142,3 (d, J=11,4 Hz, C4) ; 157,4 (d, J=246,7 Hz, C2) ; 164,2 (C=O); 164,7 (C=O).
RMN ¹⁹F (CDCl₃, 188 MHz) : -44,02 ppm.
SM (DCl/NH₃, m/z) : 384 [M+NH₄⁺].
IR (KBr, ν) : 3474 (O-H) ; 1746 (C=O) ; 1266 (C-O ester) ; 1250 (Ar-F) ; 1105 (C-O ester) ; 1044 (C-O(H)).
Pf : 74°C.
Rf : 0,25 (DCM/AcOEt/EP : 52/35/13).

### Exemple 7 :

### Benzoate de 5-fluoro-4-(hydroxyméthyl)-2-méthoxyphényle

(ce composé ne fait pas partie de cette invention).

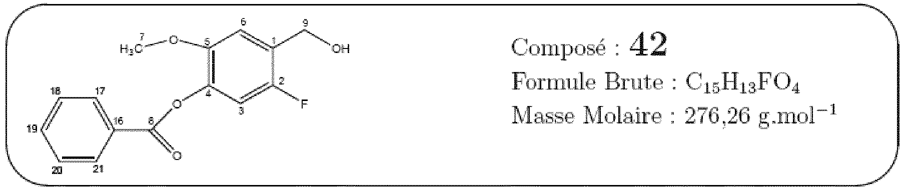

A 1,57 g (3,40 mmol ; 0,2 éq.) de chlorhydrate de chlorure d'hexabutylguanidinium (HBGC.HCl), préalablement séchés pendant 30 minutes sous pression réduite sont additionnés 3,02 g (16,40 mmol ; 1,0 éq.) de 6-fluorovératraldéhyde 7 ainsi que 4,75 ml (40,90 mmol ; 2,5 éq.) de chlorure de benzoyle. Le milieu réactionnel est chauffé à 180°C sous pression pendant 1 heure à l'aide des micro-ondes (300 watts). Il est ensuite passé sur silice avec une élution au DCM puis évaporé sous pression réduite. Le brut est mis dans 15 ml de THF. A ce mélange est ajoutée une solution aqueuse contenant 1,24 g (32,70 mmol ; 2,0 éq.) de NaBH₄ dans 15 ml d'eau. Après agitation pendant 1 heure, le milieu est extrait à l'AcOEt. La phase organique est lavée avec une solution saturée en NaHCO₃ puis avec une solution saturée en NaCl. Elle est ensuite séchée sur MgSO₄ et évaporée sous pression réduite. Le brut est purifié sur une colonne MPLC (silice ; EP/DCM/AcOEt : 45/44/11). 804 mg (18%) de benzoate de 5-fluoro-4-(hydroxyméthyl)-2-méthoxyphényle **42** sont obtenus sous la forme de paillettes blanches.
RMN ¹H (CDCl₃, 300 MHz) : 3,81 (3H, s, CH₃(7)) ; 4,76 (2H, s, CH₂(9)) ; 6,95 (1 H, d, J=6,7 Hz, H(3)) ; 7,11 (1 H, d, J=6,0 Hz, H(6)) ; 7,53 (2H, t, J=7,6 Hz, H(18), H(20)) ; 7,66 (1 H, t, J=7,5 Hz, H(19)) ; 8,22 (2H, d, J=7,5 Hz, H(17), H(21)).
RMN ¹³C (CDCl₃, 75 MHz) : 56,5 (C7) ; 58,8 (d, J=3,8 Hz, C9) ; 110,8 (d, J=25,7 Hz, C3) ; 112,3 (d, J=5,3 Hz, C6) ; 125,8 (d, J=16,6 Hz, C1) ; 128,6 (C18, C20) ; 129,0 (C16) ; 130,3 (C17, C21) ; 133,7 (C19) ; 139,3 (d, J=11,3 Hz, C4) ; 147,9 (d, J=2,3 Hz, C5) ; 153,5 (d, J=240,8 Hz, C2) ; 164,6 (C8).
RMN ¹⁹F (CDCl₃, 188 MHz) : -52,33 ppm.
SM (DCl/NH₃, m/z) : 277 [M+H₊] ; 294 [M+NH₊₄].
IR (KBr, ν) : 3289 (O-H) ; 1746 (C=O) ; 1249 (C-O ester, Cₛₚ₂-O éther et Ar-F) ; 1109 (C-O ester) ; 1059 (Cₛₚ₃-O éther).
Pf : 90°C.
Rf : 0,16 (DCM/EP/AcOEt : 52/35/13).

### Exemple 8 :

### Dibenzoate de 4-fluoro-5-(iodométhyl)-1,2-phénylène

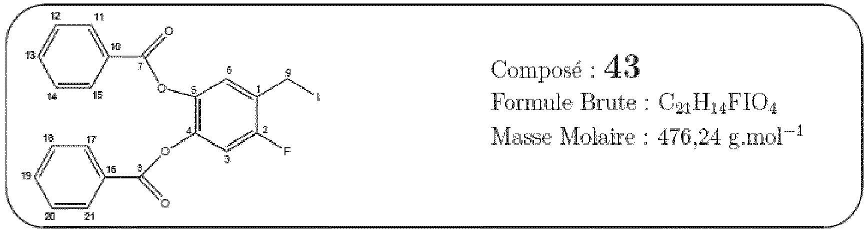

Méthode générale d'iodation avec les quantités suivantes :
PPh₃ : 143 mg (0,54 mmol ; 2,4 éq.)
I₂ : 145 mg (0,57 mmol ; 2,5 éq.)
dans 5 ml de DCM.
Dibenzoate de 4-fluoro-5-(hydroxyméthyl)-1,2-phénylène **41 :** 83 mg (0,23 mmol ; 1,0 éq.)
Imidazole : 79 mg (1,15 mmol ; 5,0 éq.)
dans 5 ml de DCM.
Rendement : 89% (MPLC (silice ; EP/DCM : 70/30)).
Aspect : huile transparente.
RMN ¹H (CDCl₃, 400 MHz) : 4,47 (2H, s, CH₂(9)) ; 7,20 (1 H, d, J=9,6 Hz, H(3)) ; 7,37-7,44 (4H, m, H(12), H(14), H(18), H(20)) ; 7,43 (1 H, d, J=7,2 Hz, H(6)) ; 7,56-7,61 (2H, m, H(13) ; H(19)) ; 8,04-8,09 (4H, m, H(11), H(15), H(17), H(21)).
RMN ¹³C (CDCl₃, 100 MHz) : -5,0 (d, J=3,3 Hz, C9) ; 112,1 (d, J=25,8 Hz, C3) ; 125,1 (d, J=4,6 Hz, C6) ; 125,3 (d, J=10,8 Hz, C1) ; 128,5 (C10, C16) ; 128,8 (C12, C14, C18, C20) ; 130,4 (C11, C15, C17, C21) ; 134,2 (C13, C19) ; 139,0 (d, J=3,7 Hz, C5) ; 143,1 (d, J=11,4 Hz, C4) ; 157,6 (d, J=250,1 Hz, C2) ; 163,9 (C=O) ; 164,3 (C=O).
RMN ¹⁹F (CDCl₃, 188 MHz) : -40,31 ppm.
SM (DCl/NH₃, m/z) : 494 [M+NH₄⁺].
IR (NaCl, ν) : 1746 (C=O) ; 1261 (C-O ester) ; 1235 (Ar-F) ; 1109 (C-O ester). Rf : 0,69 (DCM : 100%).

### Exemple 9 :

### Benzoate de 5-fluoro-4-(iodométhyl)-2-méthoxyphényle

(ce composé ne fait pas partie de cette invention).

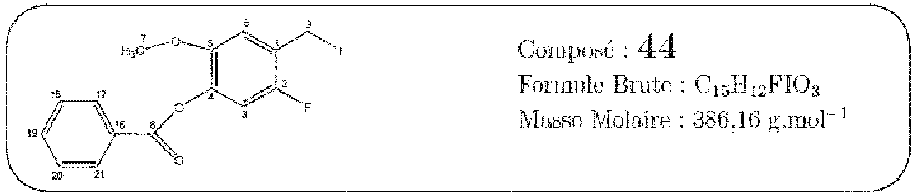

Méthode générale d'iodation avec les quantités suivantes :
PPh₃ : 281 mg (1,06 mmol ; 2,5 éq.)
I₂ : 277 mg (1,09 mmol ; 2,6 éq.)
dans 10 ml de DCM.
Benzoate 5-fluoro-4-(hydroxyméthyl)-2-méthoxyphényle **42 :** 116 mg (0,42 mmol ; 1,0 éq.)
Imidazole : 161,50 g (2,37 mmol ; 5,6 éq.)
dans 10 ml de DCM.
Rendement : 98%.
Aspect : poudre blanche.
RMN ¹H (CDCl₃, 300 MHz) : 3,73 (3H, s, CH₃(7)) ; 4,37 (2H, s, CH₂(9)) ; 6,84 (1 H, d, J=9,3 Hz, H(3)) ; 6,87 (1 H, d, J=7,2 Hz, H(6)) ; 7,44 (2H, t, J=7,6 Hz, H(18), H(20)) ; 7,57 (1 H, t, J=7,5 Hz, H(19)) ; 8,11 (2H, d, J=7,5 Hz, H(17), H(21)).
RMN ¹³C (CDCl₃, 75 MHz) : -3,6 (d, J=3,6 Hz, C9) ; 56,5 (C7) ; 111,6 (d, J=25,7 Hz, C3) ; 113,6 (d, J=4,5 Hz, C6) ; 124,2 (d, J=15,8 Hz, C1) ; 128,7 (C18, C20) ; 128,9 (C16) ; 130,4 (C17, C21) ; 134,8 (C19) ; 140,2 (d, J=10,6 Hz, C4) ; 148,0 (d, J=3,0 Hz, C5) ; 153,7 (d, J=244,6 Hz, C2) ; 164,2 (C8).
RMN ¹⁹F (CDCl₃, 282 MHz) : -123,64 ppm.
SM (DCl/NH₃, m/z) : 387 [M+H⁺] ; 404 [M+NH₄⁺] ; 421 [M+N₂H₇⁺].
IR (NaCl, ν) : 1737 (C=O ester) ; 1264 (C-O ester, Ar-F et Cₛₚ₂-O éther) ; 1115 (C-O ester) ; 1058 (Cₛₚ₃-O éther).
Pf : 124°C.
Rf : 0,13 (EP/DCM : 70/30).

### Exemple 10 :

### (S) Dibenzoate de 4-(2'-aminoéthyl-2'-(tert-butoxycarbonyl))-5-fluoro-1,2-phénylène

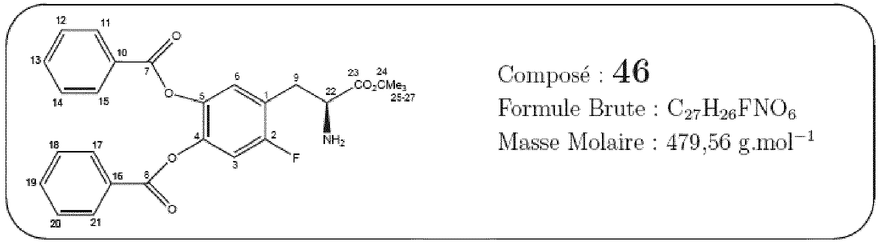

Méthode générale d'alkylation énantiosélective :

### 1^{re} étape :

Bromure de 9-(allyloxy)-1-(9-anthrylméthyl)cinchonan-1-ium **13 :** 69 mg (0,11 mmol ; 0,2 éq.).
Ester de t-butyl N-(diphénylméthylène)glycine : 173 mg (0,6 mmol ; 1,2 éq.) 5 ml de toluène.
10 ml de KOH 50%.
Dibenzoate de 4-fluoro-5-(iodométhyl)-1,2-phénylène **43** : 224 mg (0,47 mmol ; 1,0 éq.)
dans 5 ml de toluène.
20 heures sous agitation.

### 2^{e} étape :

Le brut de la 1^{re} étape d'alkylation énantiosélective (voir ci-dessus) après séparation des phases et évaporation.
5 ml THF.
15 ml d'acide citrique 15%.
3 heures sous agitation.
Masse brut : 150 mg.
Aspect : huile blanche.
RMN ¹H (CDCl₃, 500 MHz) : 1,47 (9H, s, tBu) ; 2,95 (1H, ABX, ₂J=14,0 Hz, ₃J=8,3 Hz, H(9)) ; 3,12 (1 H, ABX, ₂J=14,0 Hz, ₃J=5,7 Hz, H(9)) ; 3,73 (1 H, ABX, ₃J=5,7 Hz, ₃J=8,3 Hz H(22)) ; 7,20 (1 H, d, J=9,5 Hz, H(3)) ; 7,33 (1 H, d, J=7,0 Hz, H(6)) ; 7,37-7,42 (4H, m, H(12), H(14), H(18), H(20)) ; 7,55-7,60 (2H, m, H(13) ; H(19)) ; 8,04-8,08 (4H, m, H(11), H(15), H(17), H(21)).
RMN ¹³C (CDCl₃, 125 MHz) : 28,0 (C25-27) ; 34,3 (C9) ; 55,1 (C22) ; 81,7 (C24) ; 111,2 (d, J=27,3 Hz, C3) ; 123,4 (d, J=17,7 Hz, C1) ; 125,6 (d, J=6,0 Hz, C6) ; 128,5 (C10, C16) ; 128,6 (C12, C14, C18, C20) ; 130,2 (C11, C15, C17, C21) ; 133,8 (C13, C19) ; 138,4 (d, J=3,6 Hz, C5) ; 141,7 (d, J=11,6 Hz, C4) ; 158,3 (d, J=246,0 Hz, C2) ; 163,9 (C7) ; 164,2 (C8) ; 173,8 (C23).
RMN ¹⁹F (CDCl₃, 282 MHz) : -116,87 ppm.
SM (DCl/NH₃, m/z) : 480 [M+H⁺] ; 497 [M+NH₄⁺].
IR (NaCl, v) : 3300 (NH₂) ; 1745 (C=O ester) ; 1263 (C-O ester et Ar-F) ; 1105 (C-O ester).
Rf : 0,25 (DCM/AcOEt : 60/40).
ee : 94% (Chiracel ADH, 4,6/250 ; 1 ml/min ; hexane 0,1% DEA/IPA : 85/15 isocratique ; tR : 18,5 min, 21,9 min ; UV : 195 nm; 230 nm).

### Exemple 11 :

### (S) Benzoate de 4-(2'-aminoéthyl-2'(tert-butoxycarbonyl))-5-fluoro-2-méthoxyphényle

(ce composé ne fait pas partie de cette invention). 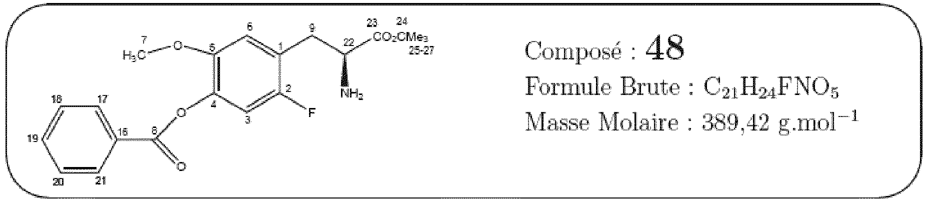 léthode générale d'alkylation énantiosélective :
Méthode générale d'alkylation énantiosélective :

### 1^{re} étape :

Bromure de 9-(allyloxy)-1-(9-anthrylméthyl)cinchonan-1-ium 13 : 56 mg (0,09 mmol ; 0,3 éq.).
Ester de t-butyl N-(diphénylméthylène)glycine : 117 mg (0,40 mmol ; 1,1 éq.) 5 ml de toluène.
10 ml de KOH 50%.
Benzoate de 5-fluoro-4-(iodométhyl)-2-méthoxyphényl **44 :** 133 mg (0,34 mmol ; 1,0 éq.) dans 5 ml de toluène.
30 heures sous agitation.

### 2^{e} étape :

Le brut de la 1^{re} étape d'alkylation énantiosélective (voir ci-dessus) après séparation des phases et évaporation.
10 ml THF.
15 ml d'acide citrique 15%.
20 heures sous agitation.
Rendement par rapport au benzoate de 5-fluoro-4-(iodométhyl)-2-méthoxyphényl **44** : 81%.
Aspect : huile blanche.
RMN ¹H (CDCl₃, 300 MHz) : 1,46 (9H, s, tBu) ; 2,92 (1 H, ABX, ₂J=14,0 Hz, ₃J=7,8 Hz, H(9)) ; 3,08 (1 H, ABX, ₂J=14,0 Hz, ₃J=6,2 Hz, H(9)) ; 3,70 (1 H, ABX, ₃J=7,8 Hz, ₃J=6,2 Hz, H(22)) ; 3,80 (3H, s, CH₃(7)) ; 6,89 (1 H, d, J=6,0 Hz, H(6)) ; 6,95 (1 H, d, J=9,0 Hz, H(3)) ; 7,52 (2H, t, J=7,5 Hz, H(18), H(20)) ; 7,66 (1 H, t, J=7,5 Hz, H(19)) ; 8,21 (2H, d, J=7,5 Hz, H(17), H(21)).
RMN ¹³C (CDCl₃, 75 MHz) : 28,0 (C25-27) ; 34,8 (C9) ; 55,2 (C22) ; 56,5 (C7) ; 81,4 (C24) ; 110,9 (d, J=27,2 Hz, C3) ; 114,8 (d, J=6,0 Hz, C6) ; 122,5 (d, J=17,4 Hz, C1) ; 128,6 (C12, C14, C18, C20) ; 129,1 (C10, C16) ; 130,3 (C11, C15, C17, C21) ; 133,6 (C13, C19) ; 138,9 (d, J=11,3 Hz, C4) ; 147,6 (d, J=3,0 Hz, C5) ; 154,7 (d, J=240,0 Hz, C2) ; 164,4 (C8) ; 174,0 (C23).
RMN ¹⁹F (CDCl₃, 282 MHz) : -125,01 ppm.
SM (DCl/NH₃, m/z) : 390 [M+H⁺] ; 407 [M+NH₄⁺].
IR (NaCl, v) : 3380 (NH₂) ; 1741 (C=O ester) ; 1261 (C-O ester, Ar-F et Cₛₚ₂-O éther) ; 1107 (C-O ester) ; 1057 (Cₛₚ₃-O éther).
Rf : 0,10 (DCM/AcOEt : 80/20).
ee : 92% (Chiracel ADH 4,6/250 ; 1 ml/min ; hexane 0,1% DEA/IPA : 95/5 iso ; tR : 9,3 min, 10,5 min ; UV : 197 nm; 228 nm; 282 nm).

### Exemple 12

### Acide (L)-2-amino-3-(2'-fluoro-4',5'-dihydroxyphényl)propanoïque ou 6-[19F] fluoro-L-Dopa

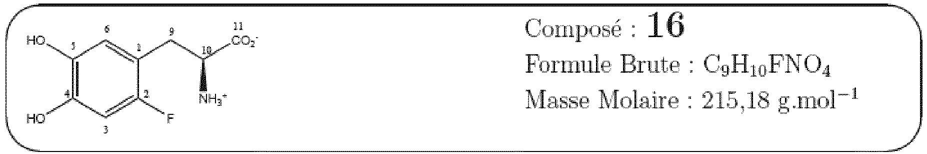

1 mmol de **46** (S) Dibenzoate de 4-(2'-aminoéthyl-2'-(tert-butoxycarbonyl))-5-fluoro-1,2-phénylène est dissous à chaud dans 5 ml d'acide chlorhydrique 3N et porté au reflux pendant 6 h. Après refroidissement, la solution est neutralisée par une solution de NH₄OH à 28%. La solution obtenue est diluée avec 4ml d'éthanol puis refroidi à -25 °C. Le précipité obtenu est lavé à l'eau froide, puis à l'éthanol froid. Après séchage, 0,6 **(46)** à 0,7 mmol du composé déprotégé est isolé sous forme d'une poudre blanche.
RMN ¹H (D2O+DCl, 300 MHz) : 2,95 (1 H, ABX, 2J=12,9 Hz, 3J=7,2 Hz, 4JH-F=0,9 Hz, H(9)) ; 3,11 (1 H, ABX, 2J=12,9 Hz, 3J=5,7 Hz, 4JH-F=0,9 Hz, H(9)) ; 4,12 (1 H, ABX, 3J=7,2 Hz, 3J=5,7 Hz, H(10)) ; 6,56 (1 H, d, J=10,8 Hz, H(3)) ; 6,61 (1 H, d, J=7,2 Hz, H(6)).
RMN ¹³C (D2O+DCl, 75 MHz) : 29,0 (C9) ; 53,9 (C10) ; 103,8 (d, J=27 Hz, C3) ; 111,9 (d, J=17,3 Hz, C1) ; 117,6 (d, J=6,0 Hz, C6) ; 140,3 (d, J=2,3 Hz, C5) ; 144,6 (d, J=11,3 Hz, C4) ; 155,0 (d, J=236,2 Hz, C2) ; 172,0 (C11).
RMN ¹⁹F (D2O+DCl, 282 MHz) : -126,10 ppm.
SM (IS, m/z) : 214 [M-H]- ; 216 [M+H]+ ; 238 [M+Na]+.
IR (NaCl, ν) : 3415 (OH) ; 3151 (NH⁺₃ asym) ; 1632 (CO⁻₂ asym) ; 1259 (Ar-F). Pf : 250°C.
[α]³⁰_{D} : +4 (c=2, 1M HCl).
ee : 77% (Chirobiotic T, 4,6/250 ; 0,8 ml/min ; EtOH/eau : 75/25 isocratique ; tR : 6,9 min, 13,7 min).

### HPLC :

- Atlantis dC 18 3µm 150x3
- Acide formique-TEA pH=5 isocratique
- Débit : 0,6 ml/min
- tR=4,4 min
- UV : 216 nm; 281 nm.

### Exemple 13

### 3-(2-fluoro-4-hydroxy-5-methoxyphenyl)alanine

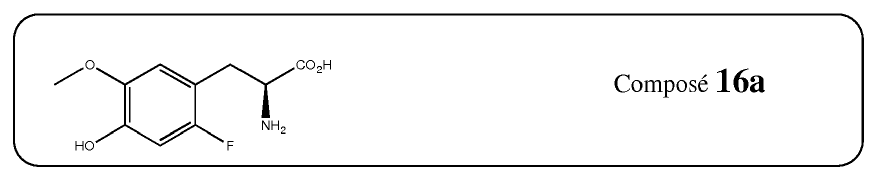

Le composé 16a a été préparé selon le même mode opératoire ci-dessus.
RMN 1 H (300 MHz, D2O+DCl) : 2,97 (1 H, ABX, *J* 13,0; 7,4 ; 1,1) ; 3,15 (1 H, ABX, *J* 13,0; 7,4; 1,1); 3,78 (3H, s) ; 4,15 (1 H, ABX, J 7,5 ; 5,4) ; 6,54 (1 H, d, J 10,6) ; 6,75 (1 H, d, *J* 7,0).
RMN 13C (75 MHz, D2O+DCl) : 28,5 ; 54,2 ; 58,1 ; 102,9 ; 112,6 (d, *J* 18); 115,7 (d, J 6) ; 1443 (d, *J 11) ;* 146 (d, *J 2) ;* 154 (d, *J*235) ; 175.
RMN 19F (282 MHz, D2O+DCl) : -125
SM (IS, m/z) : 230 [M+H]+
Pf : 246 °C

### REFERENCES

- E.S. Garnett, G. Firnau, C. Nahmias, and R. Chirakal. Striatal dopamine metabolism in living monkeys examined by positron émission tomography. Brain Res., 280 :169, 1983.
- E.S. Garnett, G. Firnau, and C. Nahmias. Dopamine visualized in the basal ganglia of living man. Nature, 305 :137-138, 1983.
- C. Lemaire, M. Guillaume, and L. Christiaens. Asymmetric synthesis of 6-[18F]fluoro-I-dopa via nca nucleophilic fluorination. J. Nucl. Med., 30 :752, 1989.
- C. Lemaire, M. Guillaume, R. Cantineau, A. Plenevaux, and L. Christiaens. An approach to the asymmetric synthesis of I-6-[18f]fluorodopa via nca nucleophilic fluorination. Appl. Radiat. Isot., 42(7) :629-635, 1991.
- C. Lemaire, S. Gillet, S. Guillouet, A. Plenevaux, J. Aerts, and A. Luxen. Highly enantioselective synthesis of no-carrier-added 6-[18F]fluoro-L-dopa by chiral phase-transfer alkylation. Eur. J. Org. Chem., 13 :2899-2904, 2004.
- F. Bourrel, S. Massou, M. Baltas, M. Bergon, M. Tafani, J.P. Esquerre, P. Tisnes, and Y. Prigent. Synthesis, nmr characterization and pharmacological evaluation of ligands derived from diprenorphine for central opioid receptors imaging. J. Phys. Org. Chem., 14(12):869-878, 2001.
- E.J. Corey, F. Xu, and M.C. Noe. A rational approach to catalytic enantioselective enolate alkylation using a structurally rigidified and defined chiral quaternary ammonium salt under phase transfer conditions. J. Am. Chem. Soc., 119 :12414-12415, 1997.
- R. A. Olofson, A. P. Lawson, and H. L. Rayle. Process for obtaining aryl esters by O-dealkylation and applications, 1994. Brevet N°US5283358.
- C. Marette, C. Larrouquet, P. Tisnès, J.-B. Delaye et E. Gras, A simple and efficient transprotection of aryl methyl ether to aryl benzoate under microwave activation, Tetrahedron Letters 47 (2006) 6947-6950.

## Revendications

1. Composé de formule (II) : 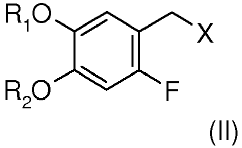 dans laquelle :
F est le ¹⁹F ou le ¹⁸F ;
R₁ représente un groupe aroyle ;
R₂ représente un groupe aroyle ;
X est Cl, Br, I, ou un groupe OSO₂R₃ ;
R₃ représente un groupe C₁-C₆ alkyle, ledit groupe alkyle étant éventuellement mono-, poly- ou perhalogéné, un groupe C₃-C₈ cycloalkyle ou un groupe C₆-C₁₂ aryle, lesdits groupes alkyle, cycloalkyle, ou aryle étant substitués par F, Cl, Br, NO₂ ou CN.

2. Procédé de préparation d'un énantiomère ou d'un mélange énantiomériquement enrichi d'un composé de formule (I) : 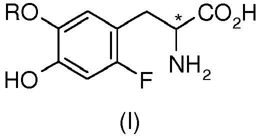 dans laquelle :
R est H ou CH₃, de préférence H ; et
F est le ¹⁹F ou le ¹⁸F, de préférence ¹⁸F,
comprenant les étapes de :
i) alkylation d'un composé de formule (II) telle que définie dans la revendication 1 par une imine de formule (III-1) : 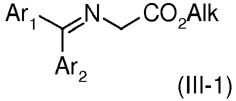 dans laquelle
Ar₁, Ar₂ sont des groupes aryles ; et
Alk représente un groupe alkyle en C₁-C₆ ;
en milieu biphasique eau/solvant organique, en présence d'un catalyseur de transfert de phase chiral (CTP*) et d'une base, ce par quoi on obtient un énantiomère ou un mélange énantiomériquement enrichi d'un composé de formule (IV-1) : 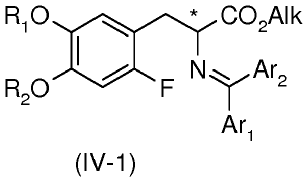
R₁ et R₂ étant tel que défini à la revendication 1
le solvant organique étant préférentiellement du toluène,
(ii) déprotection des fonctions alcool protégées par un groupement aroyle et déprotection des fonctions amine, acide carboxylique; du composé de formule (IV-1), ce par quoi on obtient un énantiomère ou un mélange énantiomériquement enrichi d'un composé de formule (I), et éventuellement
(iii) la récupération du composé de formule (I) obtenu.

3. Procédé selon la revendication 2, dans lequel le composé de formule (I) est le composé 6-F-L-Dopa de formule :

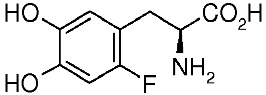

4. Procédé selon la revendication 2 ou 3, dans lequel le CTP* est choisi parmi :
- un composé de formule (A) : 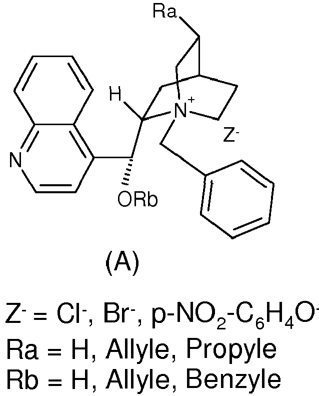
- un composé de formule (B) : 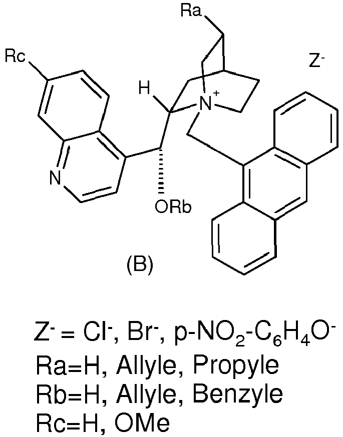
- un composé de formule (C) : 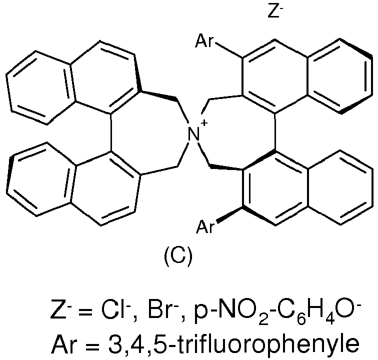
- un composé de formule (D) : 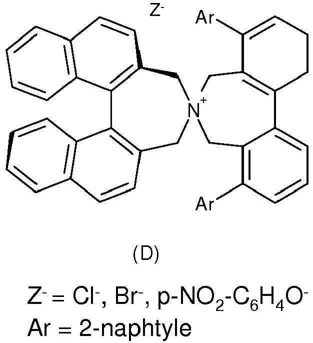 ou
- un composé de formule (E) :

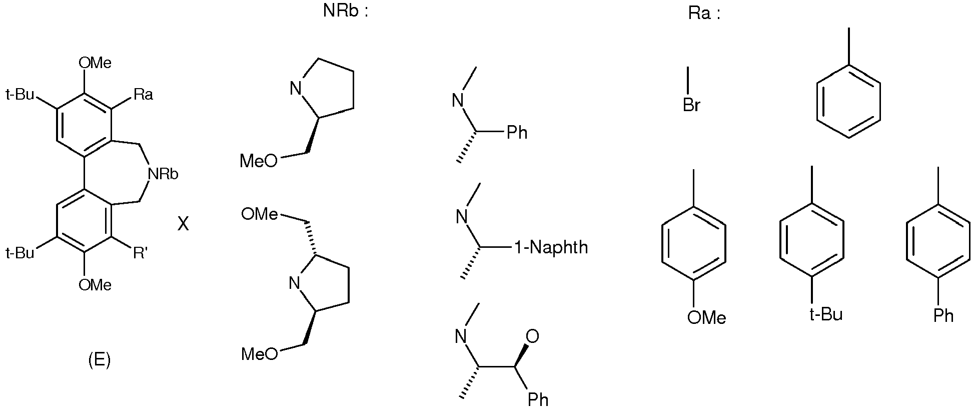

5. Procédé selon la revendication 4, dans laquelle le CTP* est le composé de formule (B1) : 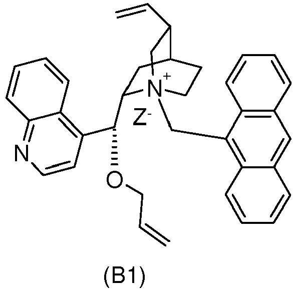 dans laquelle Z est choisi parmi Cl, Br, ou I.

6. Procédé selon l'une quelconque des revendications 2 à 5, dans lequel le composé de formule (III-1) est le composé de formule (III-1a) :

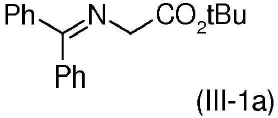

7. Procédé de préparation d'un énantiomère ou d'un mélange énantiomériquement enrichi d'un composé de formule (I) tel que défini à la revendication 2, comprenant :
(i) l'alkylation d'un composé de formule (II) tel que défini selon la revendication 1, par une imine de formule (III-2) : 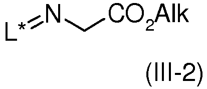 dans laquelle L* représente une copule chirale, et Alk un groupe alkyle en C₁-C₆, en présence d'une base dans un solvant organique, ce par quoi on obtient un diastéréoisomère ou un mélange diastéréoisomériquement enrichi de formule (IV-2) : 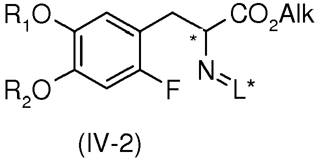 R₁ et R₂ étant tel que défini à la revendication 1
(ii) déprotection des fonctions alcool protégées par un groupement aroyle et déprotection des fonctions amine, acide carboxylique du composé de formule (IV-2), ce par quoi on obtient un énantiomère ou un mélange énantiomériquement enrichi d'un composé de formule (I), et éventuellement;
(iii) la récupération du composé de formule (I) obtenu.

8. Procédé selon la revendication 7, dans lequel l'imine de formule (III-2) est le composé de formule (III-2a) :

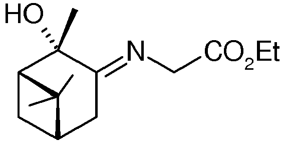

9. Procédé selon l'une quelconque des revendications 2 à 8, dans lequel l'étape ii) de déprotection comprend une hydrolyse acide du composé de formule (IV-1) ou (IV-2) en présence d'un antioxydant, ce par quoi on obtient un énantiomère ou un mélange énantiomériquement enrichi d'un composé de formule (I).

10. Procédé selon l'une quelconque des revendications 2 à 9, dans lequel le composé de formule (II) est préparé à partir d'un composé de formule (VI) : 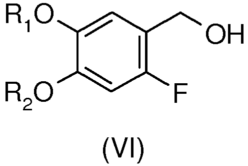 dans laquelle R₁, R₂, F sont tels que définis dans la revendication 1.

11. Procédé selon la revendication 10, dans lequel le composé de formule (II) est préparé selon un procédé comprenant la réaction du composé de formule (VI) avec PPh₃, I₂ en présence d'imidazole dans le dichlorométhane.

12. Procédé selon l'une quelconque des revendications 2 à 11, dans lequel le composé de formule (VI) est préparé à partir d'un composé de formule (VII) : 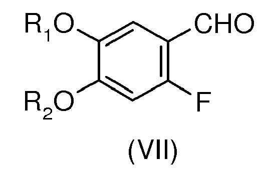 dans laquelle R₁, R₂, F sont tels que définis dans la revendication 1.

13. Procédé selon la revendication 12, dans lequel le composé de formule (VI) est préparé selon un procédé comprenant la réaction du composé de formule (VII) avec un hydrure métallique, en présence d'eau dans un solvant polaire aprotique.

14. Procédé selon l'une quelconque des revendications 2 à 13, dans lequel le composé (VII) est préparé à partir d'un composé de formule (VIII): 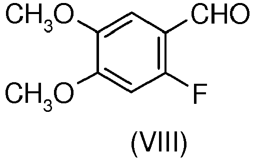 dans laquelle F est tel que définis dans la revendication 1.

15. Procédé selon la revendication 14, dans lequel le composé (VII) est préparé selon un procédé comprenant la réaction du composé de formule (VIII) avec un composé R₁Y ou R₂Y où Y représente Br, I, Cl et R₁, R₂ sont tels que définis à la revendication 1, en présence d'hexabutylguanidinium.HCl (HBGC.HCl) à titre de catalyseur dans un solvant aromatique.

16. Composé de formule (VI) : 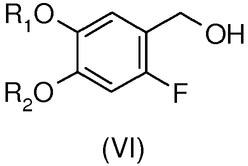 dans laquelle F, R₁ et R₂ sont tels que définis à la revendication 1.

17. Composé de formule (VII) 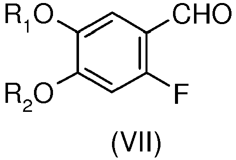 dans laquelle F, R₁ et R₂ sont tels que définis à la revendication 1, à l'exclusion du composé dans lequel R₁=R₂= Ph(CO)- et F est ¹⁹F.

## Patentansprüche

1. Verbindung der Formel (II): 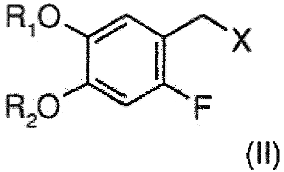 wobei:
F ¹⁹F oder ¹⁸F ist;
R₁ eine Aryl-CO-Gruppe darstellt;
R₂ eine Aryl-CO-Gruppe darstellt;
X Cl, Br, I oder eine OSO₂R₃-Gruppe ist;
R₃ eine C₁-C₆-Alkylgruppe, wobei die Alkylgruppe eventuell ein Mono-, Poly- oder Perhalogen ist, eine C₃-C₈-Cycloalkylgruppe oder eine C₆-C₁₂-Arylgruppe darstellt, wobei die Alkyl-, Cycloalkyl- oder Arylgruppen durch F, Cl, Br, NO₂ oder CN substituiert sind.

2. Verfahren zur Herstellung eines Enantiomers oder einer enantiomerisch angereicherten Mischung einer Verbindung der Formel (I): 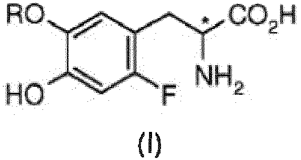 wobei:
R H oder CH₃, bevorzugt H, ist; und
F ¹⁹F oder ¹⁸F, bevorzugt ¹⁸F, ist;
umfassend die folgenden Schritte:
i) Alkylierung einer Verbindung der Formel (II), wie in Anspruch 1 definiert, durch ein Imin der Formel (III-1): 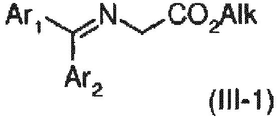 wobei:
Ar₁ und Ar₂ Arylgruppen sind; und
Alk eine C₁-C₆-Alkylgruppe darstellt;
in der Umgebung eines zweiphasigen Wassers/organischen Lösungsmittels, in Gegenwart eines chiralischen Phasentransferkatalysators (PTC*) und einer Base, wobei ein Enantiomer oder eine enantiomerisch angereicherte Mischung einer Verbindung der Formel (IV-1) erhalten wird: 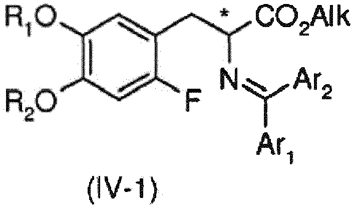
wobei R₁ und R₂ wie in Anspruch 1 definiert sind,
das organische Lösungsmittel bevorzugt Toluol ist,
(ii) Entschützung der durch eine Aryl-CO-Gruppe geschützten alkoholischen Funktionen und Entschützung der Amin- und Carbonsäurefunktionen von der Verbindung der Formel (IV-1);
wobei ein Enantiomer oder eine enantiomerisch angereicherte Mischung einer Verbindung der Formel (I) erhalten wird,
und eventuell
(iii) die Rückgewinnung einer Verbindung der Formel (I) erzielt wird.

3. Verfahren nach Anspruch 2, wobei die Verbindung der Formel (I) die Verbindung 6-F-L-Dopa gemäß der folgenden Formel ist:

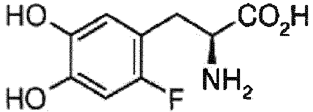

4. Verfahren nach Anspruch 2 oder 3, wobei der PTC* aus folgenden Verbindungen gewählt wird:
- einer Verbindung der Formel (A): 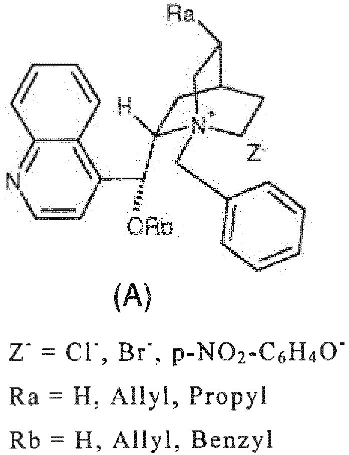
- einer Verbindung der Formel (B): 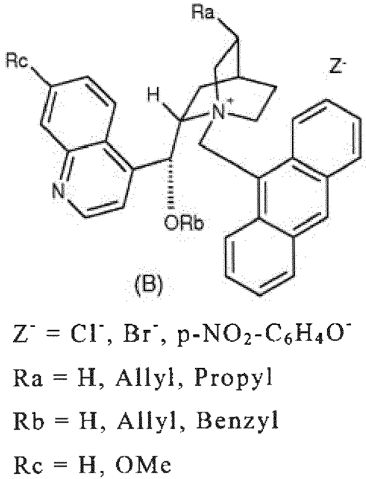
- einer Verbindung der Formel (C): 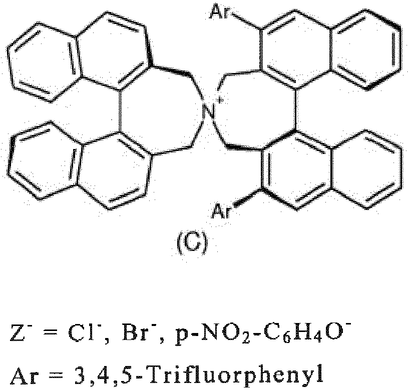
- einer Verbindung der Formel (D): 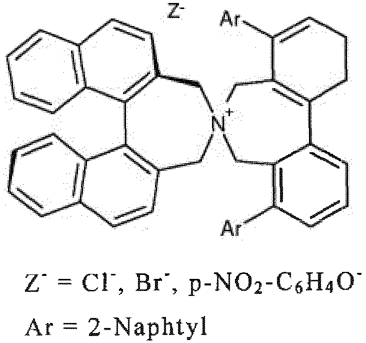 oder
- einer Verbindung der Formel (E):

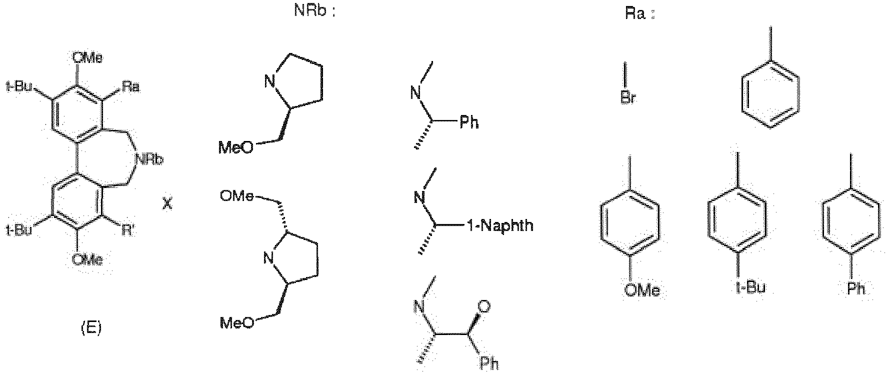

5. Verfahren nach Anspruch 4, wobei der PTC* die Verbindung der Formel (B1) ist: 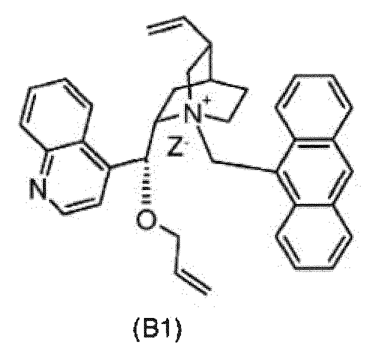 wobei Z aus Cl, Br oder I gewählt wird.

6. Verfahren nach einem der Ansprüche 2 bis 5, wobei die Verbindung der Formel (III-1) die Verbindung der Formel (III-1a) ist:

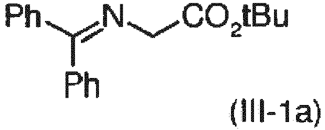

7. Verfahren zur Herstellung eines Enantiomers oder einer enantiomerisch angereicherten Mischung einer Verbindung der Formel (I), wie in Anspruch 2 definiert, umfassend:
(i) Alkylierung einer Verbindung der Formel (II) wie in Anspruch 1 definiert, durch ein Imin der Formel (III-2) 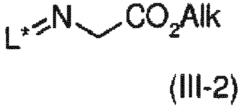 wobei L* eine chiralische Gruppe und Alk eine C₁-C₆-Alkylgruppe darstellt, in Gegenwart einer Base in einem organischen Lösungsmittel, wobei ein Diastereoisomer oder eine diastereoisomerisch angereicherte Mischung der Formel (IR-2) erhalten wird: 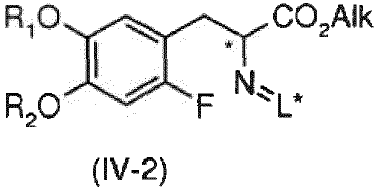 wobei R₁ und R₂ wie in Anspruch 1 definiert sind
(ii) Entschützung der durch eine Aryl-Co-Gruppe geschützten alkoholischen Funktionen und Entschützung der Amin- und Carbonsäurefunktionen
von der Verbindung der Formel (IV-2),
wobei ein Enantiomer oder eine enantiomerisch angereicherte Mischung einer Verbindung der Formel (I) erhalten wird, und eventuell
(iii) die Rückgewinnung einer Verbindung der Formel (I) erzielt wird.

8. Verfahren nach Anspruch 7, wobei das Imin der Formel (III-2) die Verbindung der Formel (III-2a) ist:

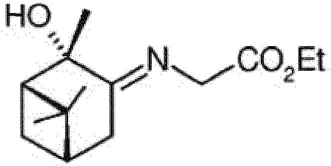

9. Verfahren nach einem der Ansprüche 2 bis 8, wobei der Schritt ii) der Entschützung eine saure Hydrolyse der Verbindung der Formel (IV-1) oder der Formel (IV-2) in Gegenwart eines Antioxidanten umfasst, wobei ein Enantiomer oder eine enantiomerisch angereicherte Mischung einer Verbindung der Formel (I) erhalten wird.

10. Verfahren nach einem der Ansprüche 2 bis 9, wobei die Verbindung der Formel (II) ausgehend von der Verbindung der Formel (VI) hergestellt wird: 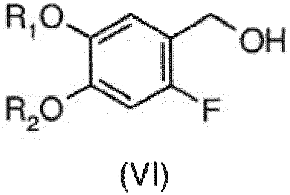 wobei R₁, R₂ und F wie in Anspruch 1 definiert sind.

11. Verfahren nach Anspruch 10, wobei die Verbindung der Formel (II) nach einem Verfahren hergestellt wird, das eine Reaktion der Verbindung der Formel (IV) mit PPh₃, I₂ in Gegenwart eines Imidazols in dem Dichlormethan umfasst.

12. Verfahren nach Anspruch 2 bis 11, wobei die Verbindung der Formel (VI) ausgehend von der Verbindung der Formel (VII) hergestellt wird: 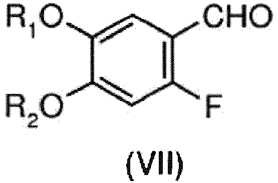 wobei R₁, R₂ und F wie in Anspruch 1 definiert sind.

13. Verfahren nach Anspruch 12, wobei die Verbindung der Formel (VI) nach einem Verfahren hergestellt wird, das die Reaktion der Verbindung der Formel (VII) mit einem Metallhydrid umfasst, in Gegenwart von Wasser in einem aprotischen polarischen Lösungsmittel.

14. Verfahren nach einem der Ansprüche 2 bis 13, wobei die Verbindung (VII) ausgehend von einer Verbindung der Formel (VIII) hergestellt wird: 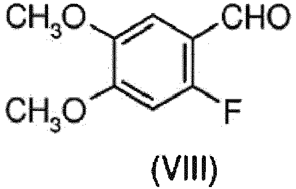 wobei F wie in Anspruch 1 definiert ist.

15. Verfahren nach Anspruch 14, wobei die Verbindung (VII) nach einem Verfahren hergestellt wird, das die Reaktion der Verbindung der Formel (VIII) mit einer Verbindung R₁Y oder R₂Y umfasst, in der Y Br, I, Cl darstellt, und R₁ und R₂ wie in Anspruch 1 definiert sind, in Gegenwart von Hexabutylguanidin.HCl (HBGC.HCl) als Katalysator in einem aromatischen Lösungsmittel.

16. Verbindung der Formel (VI): 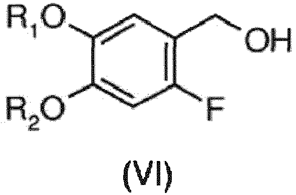 wobei F, R₁ und R₂ wie in Anspruch 1 definiert sind.

17. Verbindung der Formel (VII): 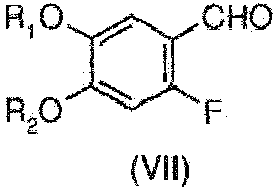 wobei F, R₁ und R₂ wie in Anspruch 1 definiert sind, mit Ausnahme der Verbindung, bei der R₁ = R₂ = Ph(CO)- und F ¹⁹F ist.

## Claims

1. Compound of formula (II): 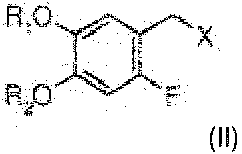 wherein:
F is ¹⁹F or ¹⁸F;
R₁ represents an aroyl group;
R₂ represents an aroyl group;
X is Cl, Br, I or a group OSO₂R₃;
R₃ represents a C₁-C₆alkyl group, said alkyl group being optionally mono-, poly- or per-halogenated, a C₃-C₆cycloalkyl group or a C₆-C₁₂aryl group, said alkyl, cycloalkyl or aryl groups being substituted by F, Cl, Br, NO₂ or CN.

2. Process for the preparation of an enantiomer or an enantiomerically enriched mixture of a compound of formula (I): 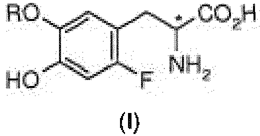 wherein:
R is H or CH₃, preferably H; and
F is ¹⁹F or ¹⁸F, preferably ¹⁸F,
comprising the steps:
i) alkylation of a compound of formula (II) as defined in claim 1 using an imine of formula (III-1): 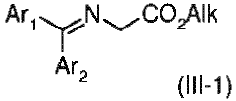 wherein
Ar₁, Ar₂ are aryl groups; and
Alk represents a C₁-C₆alkyl group;
in a water/organic solvent two-phase medium, in the presence of a chiral phase-transfer catalyst (CTP*) and a base, by which means there is obtained an enantiomer or an enantiomerically enriched mixture of a compound of formula (IV-1): 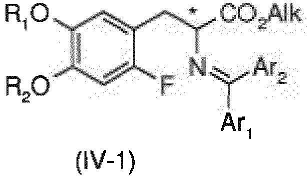 R₁ and R₂ being as defined in claim 1,
the organic solvent preferably being toluene,
(ii) deprotection of the alcohol functions protected by an aroyl group and deprotection of the amine and carboxylic acid functions
of the compound of formula (IV-1), by which means
there is obtained an enantiomer or an enantiomerically enriched mixture of a compound of formula (I),
and optionally
(iii) recovery of the compound of formula (I) obtained.

3. Process according to claim 2, wherein the compound of formula (I) is the 6-F-L-Dopa compound of formula:

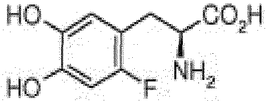

4. Process according to claim 2 or 3, wherein the CTP* is selected from:
- a compound of formula (A): 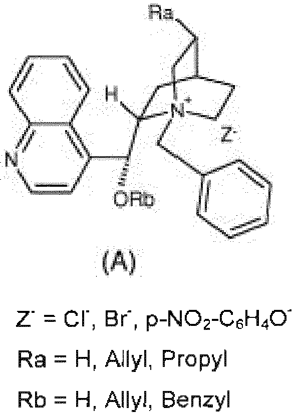
- a compound of formula (B). 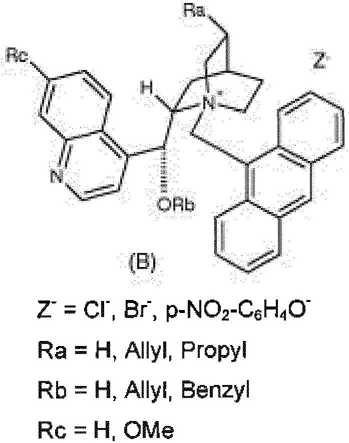
- a compound of formula (C): 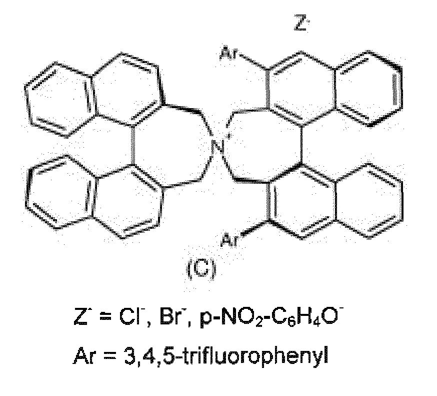
- a compound of formula (D): 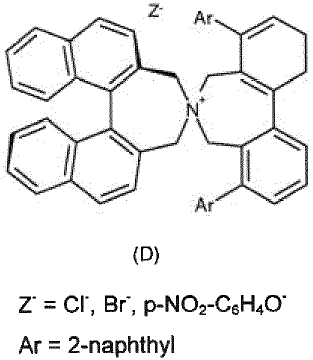 or
- a compound of formula (E):

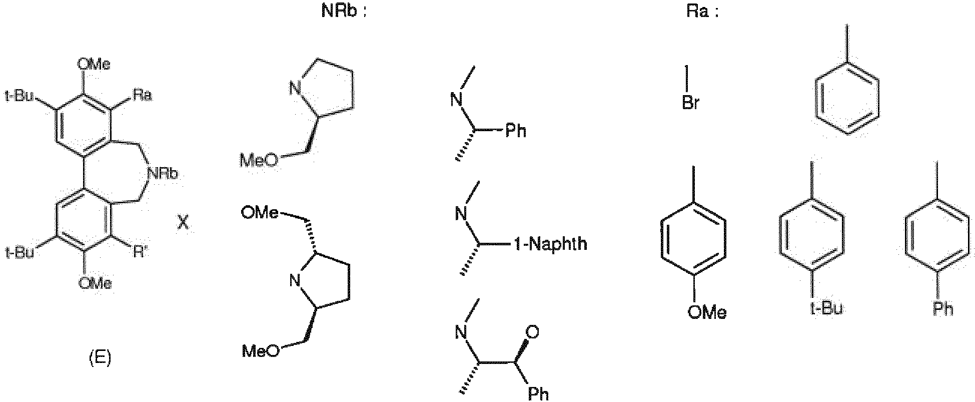

5. Process according to claim 4, wherein the CTP* is the compound of formula (B1): 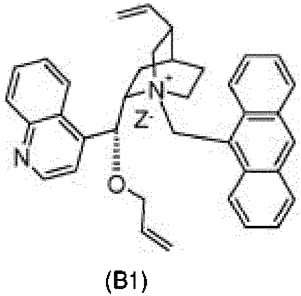 wherein Z is selected from Cl, Br or I.

6. Process according to any one of claims 2 to 5, wherein the compound of formula (III-1) is the compound of formula (III-1a):

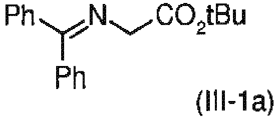

7. Process for the preparation of an enantiomer or an enantiomerically enriched mixture of a compound of formula (I) as defined in claim 2, comprising:
i) alkylation of a compound of formula (II) as defined according to claim 1 using an imine of formula (III-2): 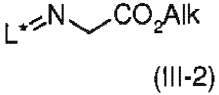 wherein L* represents a chiral copula, and Alk a C₁-C₆alkyl group, in the presence of a base in an organic solvent, by which means there is obtained a diastereoisomer or a diastereoisomerically enriched mixture of formula (IV-2): 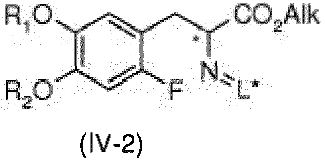 R₁ and R₂ being as defined in claim 1,
(ii) deprotection of the alcohol functions protected by an aroyl group and deprotection of the amine and carboxylic acid functions of the compound of formula (IV-2), by which means there is obtained an enantiomer or an enantiomerically enriched mixture of a compound of formula (I), and optionally;
(iii) recovery of the compound of formula (I) obtained.

8. Process according to claim 7, wherein the imine of formula (III-2) is the compound of formula (III-2a):

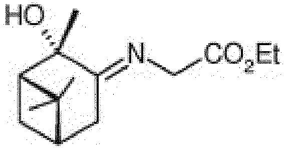

9. Process according to any one of claims 2 to 8, wherein the deprotection step ii) comprises acid hydrolysis of the compound of the compound of formula (IV-1) or (IV-2) in the presence of an antioxidant, by which means there is obtained an enantiomer or an enantiomerically enriched mixture of a compound of formula (I).

10. Process according to any one of claims 2 to 9, wherein the compound of formula (II) is prepared starting from a compound of formula (VI): 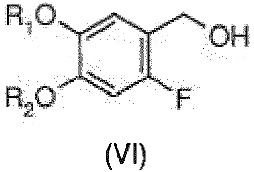 wherein R₁, R₂, F are as defined in claim 1.

11. Process according to claim 10, wherein the compound of formula (II) is prepared in accordance with a process comprising reaction of the compound of formula (VI) with PPh₃, I₂ in the presence of imidazole in dichloromethane.

12. Process according to any one of claims 2 to 11, wherein the compound of formula (VI) is prepared starting from a compound of formula (VII): 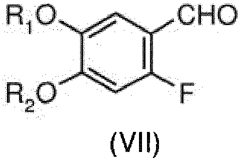 wherein R₁, R₂, F are as defined in claim 1.

13. Process according to claim 12, wherein the compound of formula (VI) is prepared in accordance with a process comprising reaction of the compound of formula (VII) with a metal hydride, in the presence of water in an aprotic polar solvent.

14. Process according to any one of claims 2 to 13, wherein compound (VII) is prepared starting from a compound of formula (VIII): 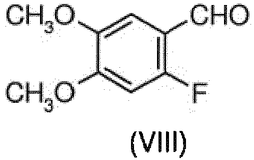 wherein F is as defined in claim 1.

15. Process according to claim 14, wherein compound (VII) is prepared in accordance with a process comprising reaction of the compound of formula (VIII) with a compound R₁Y or R₂Y wherein Y represents Br, I, Cl and R₁, R₂ are as defined in claim 1, in the presence of hexabutylguanidinium.HCl (HBGC.HCl) by way of catalyst in an aromatic solvent.

16. Compound of formula (VI): 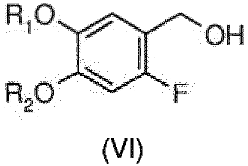 wherein F, R₁ and R₂ are as defined in claim 1.

17. Compound of formula (VII): 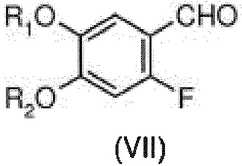 wherein F, R₁ and R₂ are as defined in claim 1, with the exclusion of the compound wherein R₁ = R₂ = PH(CO)- and F is ¹⁹F.
